# EUROPÄISCHE PATENTSCHRIFT

(11) **EP 1 414 507 B1**
(45) Veröffentlichungstag und Bekanntmachung des Hinweises auf die Patenterteilung: **30.08.2006**
(21) Anmeldenummer: 02745039.4
(22) Anmeldetag: 22.07.2002
(51) Int. Cl.: A61M 5/00, A61M 5/315

(54) **VERABREICHUNGSGERÄT MIT VERDREHSICHERUNG**
ADMINISTRATION DEVICE SECURED AGAINST ROTATION
APPAREIL D'ADMINISTRATION DE PRODUITS, MUNI D'UN SYSTEME DE BLOCAGE EN ROTATION

(30) Priorität: 30.07.2001 DE 20112501 U; 21.12.2001 DE 10163328
(43) Veröffentlichungstag der Anmeldung: 06.05.2004
(73) Patentinhaber: TecPharma Licensing AG, 3401 Burgdorf (CH)
(72) Erfinder: KIRCHHOFER, Fritz, CH-3454 Sumiswald (CH); SCHÄR, Michael, CH-4543 Deitingen (CH)
(74) Vertreter: Schwabe - Sandmair - Marx
(86) Internationale Anmeldenummer: PCT/CH2002/000413
(87) Internationale Veröffentlichungsnummer: WO 2003/011374

(56) Entgegenhaltungen:
- EP-A- 0 594 349
- EP-A- 1 095 668
- WO-A-97/17095
- DE-A- 4 425 763
- US-A- 4 592 745
- US-A- 4 973 318
- US-A- 5 611 783
- US-B1- 6 221 046

## Beschreibung

Die Erfindung betrifft ein Verabreichungsgerät für eine dosierte Verabreichung eines fluiden Produkts. Bevorzugte Beispiele erfindungsgemäßer Geräte sind Injektionsgeräte, insbesondere Injektionspens. Besonders bevorzugte Beispiele sind semidisposable Pens. Das Gerät kann auch einen Dosierteil eines Inhalationsgeräts oder eines Geräts für eine orale Einnahme oder eine anders geartete Art der Verabreichung eines fluiden Produkts bilden.

In der Verabreichung von Produkten, insbesondere in medizinischen Anwendungen, kommt der genauen Dosierung des Produkts eine große Bedeutung zu. Bei Verabreichungsgeräten, wie Injektionsgeräte sie typischerweise darstellen, wird im Allgemeinen die Dosierung mit Hilfe eines Dosiseinstellglieds vorgenommen, das mit einer Fördervorrichtung in einem Eingriff ist. Bei solchen Geräten können Probleme dann entstehen, wenn durch eine Dosierbewegung des Dosiseinstellglieds aufgrund des Eingriffs eine Reaktionsbewegung der Fördervorrichtung hervorgerufen wird, insbesondere wenn solch eine Reaktionsbewegung im Hinblick auf die korrekte Dosierung vermieden werden sollte.

Die EP 0 549 349 B1 beschreibt eine Spritze mit einer Vorrichtung zum Schutz gegen Wiederbefiillung. Zum Ausschütten der Flüssigkeit weist die Spritze eine Kolbenstange auf, über die ein Kolben in Ausschüttrichtung gedrückt werden kann. Die Kolbenstange besitzt ein Außengewinde und ist über eine Mutter mit einem Dosierknopf verbunden. Durch eine Drehung des Dosierknopfes kann die Menge der abzugebenden Flüssigkeitsmenge eingestellt werden. Um nach der Abgabe der Flüssigkeit zu verhindern, dass der Dosierknopf im die der Aufdosierungsrichtung entgegengesetzte Richtung gedreht wird, weist die Spritze der EP 0 549 349 B1 eine Sperreinrichtung auf, die ein Verdrehen der Dosiereinrichtung zum Einstellen einer neuen Abgabedosis zulässt, eine Drehung in die entgegengesetzte Richtung aber verhindert.

Es ist eine Aufgabe der Erfindung, bei einem Verabreichungsgerät, das eine Fördervorrichtung und ein zum Zwecke der Dosisauswahl mit der Fördervorrichtung gekoppeltes Dosiseinstellglied aufweist, die Exaktheit der Dosierung zu verbessern.

Die Erfindung betrifft ein Verabreichungsgerät für eine dosierte Verabreichung eines fluiden Produkts, das ein Gehäuse mit einem Reservoir für das Produkt, eine Fördervorrichtung, ein mit der Fördervorrichtung mechanisch gekoppeltes Dosiseinstellglied und einen Anschlag für das Dosiseinstellglied umfasst. Die Fördervorrichtung wird von einer Abtriebsvorrichtung und einer Antriebsvorrichtung gebildet. Die Abtriebsvorrichtung wird von dem Gehäuse so gelagert, dass sie eine Ausschüttbewegung in Form eines Ausschütthubs in eine Vorschubrichtung entlang einer Translationsachse ausführen kann, um eine mit Hilfe des Dosiseinstellglieds zuvor ausgewählte Produktdosis auszuschütten. Mit Hilfe der Antriebsvorrichtung wird die Ausschüttbewegung der Abtriebsvorrichtung bewirkt, d.h. die Antriebsvorrichtung und die Abtriebsvorrichtung sind entsprechend gekoppelt. Das Dosiseinstellglied ist mit der Abtriebsvorrichtung so gekoppelt, dass eine rotatorische Dosierbewegung, die das Dosiseinstellglied und die Abtriebsvorrichtung um die Translationsachse relativ zueinander ausführen, zwangsweise eine translatorische Dosierbewegung des Dosiseinstellglieds entlang der Translationsachse relativ zu der Abtriebsvorrichtung und dem Gehäuse bewirkt.

Ferner weist das Verabreichungsgerät einen Translationsanschlag für das Dosiseinstellglied auf, dem das Dosiseinstellglied in einer axialen Endposition axial zugewandt gegenüberliegt. Der Translationsanschlag begrenzt somit die Bewegungsmöglichkeit des Dosiseinstellglieds entlang der Translationsachse in eine Richtung. Die axiale Endposition des Dosiseinstellglieds entspricht daher entweder einer auswählbaren Maximaldosis oder Minimaldosis, welche die Nulldosis sein kann. Der Translationsanschlag kann dementsprechend ein in Bezug auf die Vorschubrichtung vorderer Translationsanschlag oder ein hinterer Translationsanschlag sein.

Führt das Dosiseinstellglied eine rotatorische Dosierbewegung aus, ist aber aus irgendeinem Grunde an der Ausführung der aus der Kopplung mit der Abtriebsvorrichtung sich ergebenden translatorischen Dosierbewegung gehindert, so wird die Abtriebsvorrichtung zur Ausführung einer axialen Reaktionsbewegung gezwungen, wenn sie nicht an der Ausführung solch einer Reaktionsbewegung gehindert ist. Eine Blockierung jeglicher Reaktionsbewegung würde jedoch entweder zu einer Beschädigung der Blockierung oder zu einer Beschädigung der Kopplung zwischen der Abtriebsvorrichtung und dem Dosiseinstellglied führen. Zu solch einer Situation kann es insbesondere dann kommen, wenn das Dosiseinstellglied die genannte axiale Endposition zu dem Translationsanschlag einnimmt.

Um das Auftreten unzulässig hoher Kräfte zu verhindern, die zu Beschädigungen oder einer unerwünschten Reaktionsbewegung der Abtriebsvorrichtung führen könnten, ist nach der Erfindung eine Verdrehsicherung vorgesehen. Die Verdrehsicherung wirkt nur in der axialen Endposition des Dosiseinstellglieds und lässt in der Endposition die rotatorische Dosierbewegung des Dosiseinstellglieds nur in eine erste Drehrichtung zu, indem sie die rotatorische Dosierbewegung in die andere, zweite Drehrichtung blockiert. Es wird selbstverständlich diejenige rotatorische Dosierbewegung blockiert, die bei Nichtblockierung dazu führen würde, dass das Dosiseinstellglied axial gegen den Translationsanschlag gepresst wird. Ohne die erfindungsgemäße Verdrehsicherung würde im Falle der Ausführung der rotatorischen Dosierbewegung in die zweite Drehrichtung bei gleichzeitiger Blockierung der zwangweisen Translationsbewegung durch den Translationsanschlag das Dosiseinstellglied mit zunehmender Kraft gegen den Translationsanschlag gepresst werden. Die erfindungsgemäße Verdrehsicherung verhindert jedoch, dass solch eine Presskraft bis auf einen unzulässigen Wert ansteigen kann oder überhaupt erst entsteht.

Die Kopplung zwischen der Abtriebsvorrichtung und dem Dosiseinstellglied wird vorzugsweise durch einen Eingriff verwirklicht, in dem die Abtriebsvorrichtung unmittelbar mit dem Dosiseinstellglied ist. Des Weiteren bewirkt die Kopplung vorzugsweise, dass die Abtriebsvorrichtung und das Dosiseinstellglied in die Vorschubrichtung nur gemeinsam bewegt werden können. Falls die Antriebsvorrichtung auf das Dosiseinstellglied wirkt, wie dies bevorzugt wird, so nimmt das Dosiseinstellglied die Abtriebsvorrichtung in die Vorschubrichtung mit. Falls die Antriebsvorrichtung auf die Abtriebsvorrichtung wirkt, nimmt diese das Dosiseinstellglied mit.

Eine Relativbewegung zwischen der Abtriebsvorrichtung und dem Dosiseinstellglied entlang der Translationsachse wird durch die rotatorische Dosierbewegung zwangweise bewirkt. Die Kopplung kann insbesondere ein Schraubgelenk umfassen oder vorteilhafterweise allein von einem einzigen Schraubgelenk gebildet werden. In der besonders bevorzugten Ausführung wird das Schraubgelenk durch einen unmittelbaren Gewindeeingriff gebildet, wobei die Gewindeachse der ineianandergreifenden Gewinde von Antriebsvorrichtung und Dosiseinstellglied am zweckmäßigsten mit der Translationsachse zusammenfällt.

In Bezug auf die Vorschubrichtung kann der Translationsanschlag ein vorderer Anschlag sein, der die gemeinsame Bewegung der Abtriebsvorrichtung und des Dosiseinstellglieds in die Vorschubrichtung begrenzt und dieser Funktion wegen im Folgenden als Ausschüttanschlag bezeichnet wird. Solch ein Ausschüttanschlag wird vorzugsweise unmittelbar von dem Gehäuse gebildet oder ist mit dem Gehäuse steif verbunden oder wird von dem Gehäuse axial nicht beweglich gelagert. Stattdessen kann der Translationsanschlag, bezogen auf die Vorschubrichtung, aber auch ein hinterer Anschlag sein, der die translatorische Dosierbewegung des Dosiseinstellglieds begrenzt. Besonders bevorzugten Ausführungen entspricht es, dass ein Ausschüttanschlag und ein hinterer Translationsanschlag in Kombination vorgesehen sind und das Dosiseinstellglied in den in diesem Fall zwei axialen Endpositionen dem jeweiligen Translationsanschlag axial gegenüberliegt, d.h. dass das Dosiseinstellglied zu beiden Translationsanschlägen den Gegenanschlag bildet. Während der Ausschüttanschlag die Bewegung der Abtriebsvorrichtung und des Dosiseinstellglieds durch einen echten Kontakt begrenzt, ist dies für den hinteren Translationsanschlag nicht unbedingt erforderlich. Die erfindungsgemäße Verdrehsicherung kann durchaus so ausgebildet sein, dass eine auf den hinteren Translationsanschlag zu gerichtete translatorische Dosierbewegung des Dosiseinstellglieds bereits blockiert wird, bevor das Dosiseinstellglied axial gegen den hinteren Translationsanschlag stoßen kann. Die Formulierung in den Ansprüchen, nämlich dass das Dosiseinstellglied in seiner axialen Endposition dem Translationsanschlag axial zugewandt gegenüberliegt, soll zum einen den Zustand beschreiben, dass ein Kontakt mit einer Kraft in axialer Richtung stattfindet, und zum anderen den Zustand, dass die Verdrehsicherung zur Wirkung kommt bevor solch ein Kontakt stattfinden kann.

Ist der Translationsanschlag ein hinterer Anschlag, so wird er in einer bevorzugten Ausführung ebenfalls von dem Gehäuse unmittelbar gebildet oder axial nicht beweglich gelagert. In einer anderen Ausführung bildet die Antriebsvorrichtung unmittelbar einen hinteren Translationsanschlag, oder es wird ein hinterer Translationsanschlag von der Antriebsvorrichtung so gelagert, dass er relativ zu der Antriebsvorrichtung axial nicht beweglich ist.

In einer Ausführung, in der das Dosiseinstellglied relativ zu dem Gehäuse um die Translationsachse nicht drehbar und stattdessen die Abtriebsvorrichtung relativ zu dem Gehäuse und dem Dosiseinstellglied für die Ausführung der rotatorischen Dosierbewegung um die Translationsachse dreht, kann die Verdrehsicherung entweder zwischen dem Dosiseinstellglied und einem Übertragungsglied oder zwischen dem Dosiseinstellglied und der Antriebsvorrichtung gebildet werden. Das Übertragungsglied ist mit der Abtriebsvorrichtung verdrehgesichert und mit dem Gehäuse axial nicht beweglich verbunden. Vorzugsweise bildet eine Blockiereinrichtung das Übertragungsglied, die dazu dient, eine Bewegung der Abtriebsvorrichtung gegen die Vorschubrichtung relativ zu dem Gehäuse zu verhindern. Die Antriebsvorrichtung ist in dieser Ausführung verdrehgesichert mit der Abtriebsvorrichtung verbunden, aber relativ zu der Abtriebsvorrichtung axial bewegbar, um zum einen die rotatorische Dosierbewegung und zum anderen den Ausschütthub zu bewirken.

In einer bevorzugten zweiten Ausführung, in der das Dosiseinstellglied für die Ausführung der rotatorischen Dosierbewegung relativ zu dem Gehäuse und relativ zu der Abtriebsvorrichtung um die Translationsachse drehbar und die Abtriebsvorrichtung vorzugsweise relativ zu dem Gehäuse nicht drehbar ist, wird die Verdrehsicherung zwischen dem Dosiseinstellglied und dem Gehäuse gebildet.

Die Verdrehsicherung kann zwar grundsätzlich durch einen Kraftschluss bewirkt werden, bevorzugt beruht die Verdrehsicherung jedoch auf einem Formschluss. Dementsprechend umfasst die Verdrehsicherung wenigstens zwei Verdrehanschläge, die für diejenige Drehrichtung der rotatorischen Dosierbewegung, die blockiert werden soll, einander zugewandte Anschlagflächen bilden, die in einen gegenseitigen Anschlag gelangen. Andererseits sind die wenigstens zwei zusammenwirkenden Verdrehanschläge so ausgebildet, dass sie die rotatorische Dosierbewegung in die andere Drehrichtung zulassen und vorzugsweise überhaupt nicht behindern. Grundsätzlich wäre es möglich, die zusammenwirkenden Verdrehanschläge in Bezug auf die Drehrichtung, die zugelassen werden soll, elastisch nachgiebig zu gestalten. Bevorzugt wird jedoch, dass die Verdrehanschläge einerseits und die Übertragung der rotatorischen Dosierbewegung in die translatorische Dosierbewegung andererseits so aufeinander abgestimmt sind, dass durch die nicht blockierte rotatorische Dosierbewegung, die für die Zwecke der Blockierung zusammenwirkenden Verdrehanschläge so rasch auseinanderbewegt werden, dass sie die zuzulassende rotatorische Dosierbewegung nicht behindern können. Am einfachsten wird dieses Ziel dadurch erreicht, dass die axiale Erstreckung der zusammenwirkenden Verdrehanschläge auf die Übertragung der rotatorischen Dosierbewegung in die translatorische Dosierbewegung abgestimmt ist.

Die für die Blockierung zusammenwirkenden Verdrehanschläge können an radial aneinander zugewandten Mantelflächen des Dosiseinstellglieds und desjenigen Körpers gebildet sein, der zusammen mit dem Dosiseinstellglied die zusammenwirkenden Verdrehanschläge bildet.

In bevorzugter Ausführung bilden jedoch das Dosiseinstellglied und der Translationsanschlag an axial einander zugewandten Stirnflächen je wenigstens einen Verdrehanschlag. Die derart gebildeten wenigstens zwei Verdrehanschläge gelangen in der axialen Endposition des Dosiseinstellglieds gegeneinander in Anschlag, um die rotatorische Dosierbewegung in die eine Drehrichtung zu blockieren. Die zusammenwirkenden Verdrehanschläge können als axial aufeinander zu ragende Vorsprünge gebildet sein. Es kann jedoch auch nur einer der zusammenwirkenden Verdrehanschläge ein Vorsprung sein, während der andere von einer Ausnehmung gebildet wird, in die der Vorsprung in der axialen Endposition des Dosiseinstellglieds hineinragt.

Falls die Dosisauswahl in diskreten Schritten vorgenommen wird und die rotatorische Dosierbewegung zwischen diskreten Drehwinkelpositionen, vorzugsweise Drehwinkelrastpositionen, stattfindet, so sind die zusammenwirkenden Verdrehanschläge vorzugsweise so angeordnet, dass sie gegeneinander auf Anschlag liegen oder unmittelbar vor ihrer gegenseitigen Anschlagposition sich befinden, wenn das Dosiseinstellglied und die Abtriebsvorrichtung relativ zueinander die diskreten Drehwinkelpositionen einnehmen. Auf diese Weise wird die unerwünschte rotatorische Drehbewegung besonders früh blockiert. Falls die zusammenwirkenden Verdrehanschläge von einem Vorsprung und einer Ausnehmung gebildet werden, wird durch solch eine Abstimmung ermöglicht, dass der Vorsprung in der axialen Endposition des Dosiseinstellglieds vollkommen in der Ausnehmung aufgenommen ist.

Wenn die Produktausschüttung wie bevorzugt durch einen Kolben vorgenommen wird, der in dem Reservoir in die Vorschubrichtung auf einen Auslass des Reservoirs zu vorgeschoben wird, bilden der Kolben und eine Kolbenstange die Abtriebsvorrichtung der Fördervorrichtung. Die Kolbenstange kann mit dem Kolben fest, d.h. ständig, verbunden sein, worunter auch eine einstückige Ausbildung von Kolben und Kolbenstange verstanden werden soll. In bevorzugter Ausführung sind der Kolben und die Kolbenstange jedoch als separate Bauteile ausgeführt, und es drückt zum Zwecke der Produktausschüttung die Kolbenstange mit einem vorderen Ende gegen eine Rückseite des Kolbens.

Die Antriebsvorrichtung ist vorzugsweise zu einer Dosier- und Antriebsvorrichtung weiterentwickelt, die bei der Dosisauswahl mitwirkt und relativ zu dem Gehäuse axial translatorisch und um die Translationsachse rotatorisch bewegbar ist. Die Dosier- und Antriebsvorrichtung ist in bevorzugten Ausführungen entweder mit der Abtriebsvorrichtung oder mit dem Dosiseinstellglied in Bezug auf die Translationsachse verdrehgesichert verbunden, vorzugsweise durch unmittelbaren Eingriff, um die rotatorische Bewegung der Dosier- und Antriebsvorrichtung unmittelbar in die rotatorische Dosierbewegung umzuwandeln.

Die Abtriebsvorrichtung, das Dosiseinstellglied und die Dosier- und Antriebsvorrichtung können durch einen unmittelbaren paarweisen Eingriff von je zwei dieser Komponenten oder Baugruppen ohne Zwischenschaltung von Übertragungsgliedern miteinander verbunden sein, wie dies bevorzugt wird. Eine Zwischenschaltung von einem oder mehreren Übertragungsgliedern ist grundsätzlich aber auch denkbar.

Die Dosier- und Antriebsvorrichtung kann manuell, halb automatisch oder voll automatisch arbeiten. Im ersten Fall wird sowohl die rotatorische Dosierbewegung als auch die translatorische Ausschüttbewegung manuell ausgeführt. Im zweiten Fall wird entweder die Dosierbewegung oder die Ausschüttbewegung manuell ausgeführt, und die andere Bewegung wird motorisch oder mittels einer anderen Art der Kraftbeaufschlagung, beispielsweise mittels Federkraft bewirkt, wenn der Verwender die entsprechende Bewegung durch einen Betätigungshandgriff ausgelöst hat. Im dritten Fall, der vollautomatischen Dosier- und Antriebsvorrichtung werden die Dosierbewegung und die Ausschüttbewegung motorisch oder mittels einer anderen Kraft, beispielsweise Federkraft, bewirkt. Es wird in diesem Fall manuell lediglich die Dosis ausgewählt, beispielsweise mittels einer oder mehreren Tasten, und es wird die Ausschüttbewegung ebenfalls durch einen eigenen, entsprechenden Betätigungshandgriff des Verwenders ausgelöst. In den meisten Ausführungen ist das erfindungsgemäße Verabreichungsgerät mit einer manuellen Dosier- und Antriebsvorrichtung ausgerüstet, die dann als Dosier- und Betätigungsvorrichtung bezeichnet wird. Soweit daher von einer Dosier- und Betätigungsvorrichtung die Rede ist, wird damit die manuelle Ausführung bezeichnet. Soweit von einer Dosier- und Antriebsvorrichtung die Rede ist, soll hierdurch keine Beschränkung in Bezug auf manuell, halb automatisch oder voll automatisch vorgenommen werden, sondern es soll jede der Ausführungen umfasst sein. Der Begriff "Dosier- und Betätigungsmodul" wird jedoch im Zusammenhang mit sämtlichen Ausführungen der Dosier- und Antriebsvorrichtung verwendet.

Die Dosier- und Antriebsvorrichtung kann ein Dosierelement, das die Dosierbewegung ausführt, und separat ein Antriebselement aufweisen, das die Ausschüttbewegung ausführt. Vorzugsweise werden die Dosierbewegung und Ausschüttbewegung jedoch von dem gleichen Körper der Dosier- und Antriebsvorrichtung ausgeführt, der im Folgenden daher auch als Dosier- und Antriebselement oder als Dosier- und Betätigungselement bezeichnet wird.

Bei dem Produkt handelt es sich vorzugsweise um ein Fluid, besonders bevorzugt eine Flüssigkeit, für eine medizinische, therapeutische, diagnostische, pharmazeutische oder kosmetische Anwendung. Das Produkt kann beispielsweise Insulin, ein Wachstumshormon oder auch dünn- bis dickflüssige, breiförmige Nahrung sein. Das Verabreichungsgerät wird vorzugsweise in solchen Verwendungen eingesetzt, in denen ein Verwender sich das Produkt selbst verabreicht, wie es beispielsweise in der Diabetestherapie üblich ist. Die Verwendung im stationären und ambulanten Bereich durch geschultes Personal soll jedoch nicht ausgeschlossen sein.

Die Produktverabreichung kann bei einem Injektionsgerät mittels Injektionskanüle oder beispielsweise einer Düse für nadellose Injektionen erfolgen. Die Injektion oder Infusion kann insbesondere subkutan oder venös vorgenommen werden, oder auch intramuskulär. Im Falle der Verabreichung per Inhalation kann die ausgewählte Produktdosis aus dem Reservoir beispielsweise in eine Kammer des Inhalationsgeräts ausgeschüttet und mittels einer Zerstäubungseinrichtung für die Inhalation zerstäubt werden. Denkbar ist ferner eine orale Einnahme oder eine Verabreichung über die Speiseröhre, um nur einige Beispiele der Verabreichung zu nennen.

Besonders bevorzugt ist das Verabreichungsgerät semidisposable. In diesem Fall ist der vordere Gehäuseabschnitt Träger eines Reservoirmoduls, das nach Entleerung des Reservoirs entsorgt oder in einen Kreislauf zurückgeführt wird, und der hintere Gehäuseabschnitt ist Träger eines Dosier- und Betätigungsmoduls, das in Verbindung mit einem neuen Reservoirmodul wiederholt verwendbar ist. Da das Reservoirmodul als Verbrauchsmodul auch separat gehandelt wird, ist es auch separat Gegenstand der Erfindung. Auch das Dosier- und Betätigungsmodul kann separat Gegenstand der Erfindung sein. Ebenso bildet ein System aus einem Verabreichungsgerät und wenigstens einem Reservoirmodul, das das Reservoirmodul des Geräts nach Verbrauch ersetzen kann, einen Gegenstand der Erfindung. Das Konzept des zweigeteilten Verabreichungsgeräts in einen für die nur einmalige Verwendung vorgesehenen Teil und einen für die wiederholte Verwendung vorgesehenen Teil (semidisposable) ist vorteilhaft insbesondere für Injektionspens, aber desweiteren auch für beispielsweise Inhalationsgeräte oder Geräte für die orale Einnahme eines Produkts oder eine künstliche Ernährung.

In den Unteransprüchen werden weitere bevorzugte Ausgestaltungen der Erfindung beschrieben, wobei Merkmale die nur in Bezug auf das Verabreichungsgerät oder nur in Bezug auf ein Reservoirmodul oder ein Dosier- und Betätigungsmodul beansprucht sind, auch bevorzugte Merkmale in Bezug auf den jeweils anderen Anspruchsgegenstand sind.

Ausführungsbeispiele der Erfindung werden nachfolgend anhand von Figuren beschrieben. An den Ausführungsbeispielen offenbar werdende Merkmale bilden je einzeln und in jeder Merkmalskombination die Gegenstände der Ansprüche vorteilhaft weiter. Auch Merkmale, die nur an dem einen Beispiel offenbart sind, bilden das jeweils andere Beispiel weiter oder zeigen eine Alternative auf, soweit nichts Gegenteiliges offenbart wird oder nur der Fall sein kann. Es zeigen:
- Figur 1: zwei Teile eines Reservoirmoduls nach einem ersten Ausführungsbeispiel,
- Figur 2: das aus den zwei Teilen der Figur 1 erhaltene Reservoirmodul,

- Figur 3: ein das Reservoirmodul der Figur 2 umfassendes Injektionsgerät nach dem ersten Ausführungsbeispiel in einem Längsschnitt,
- Figur 4: einen Teil des Injektionsgeräts der Figur 3,
- Figur 5: einen Mechanikhalter des Reservoirmoduls in einem Längsschnitt und zwei Ansichten,
- Figur 6: eine von dem Mechanikhalter gelagerte Blockiereinrichtung für eine Kolbenstange,
- Figur 7: eine Kolbenstange in einem Längsschnitt und einer Stirnansicht,
- Figur 8: eine Verriegelungssperre in einem Längsschnitt, einer Ansicht und einer Draufsicht,
- Figur 9: ein zweites Ausführungsbeispiel eines Injektionsgeräts,
- Figur 10: den Querschnitt A-A der Figur 9,
- Figur 11: den Querschnitt B-B der Figur 9,
- Figur 12: den Querschnitt C-C der Figur 9,
- Figur 13: den Querschnitt D-D der Figur 9,
- Figur 14: den Mechanikhalter des zweiten Ausführungsbeispiels in einer perspektivischen Darstellung,
- Figur 15: den Mechanikhalter der Figur 14 in einer Ansicht,
- Figur 16: den Querschnitt A-A der Figur 15,
- Figur 17: das Dosiseinstellglied des zweiten Ausführungsbeispiels in einer perspektivischen Darstellung,
- Figur 18: das Dosiseinstellglied der Figur 17 in einem Längsschnitt,
- Figur 19: das Dosiseinstellglied der Figur 17 in einer Ansicht,
- Figur 20: das Dosiseinstellglied der Figur 17 in einer Draufsicht,
- Figur 21: einen Teil des Injektionsgeräts nach Figur 3 und
- Figur 22: einen Teil des Injektionsgeräts nach Figur 9.

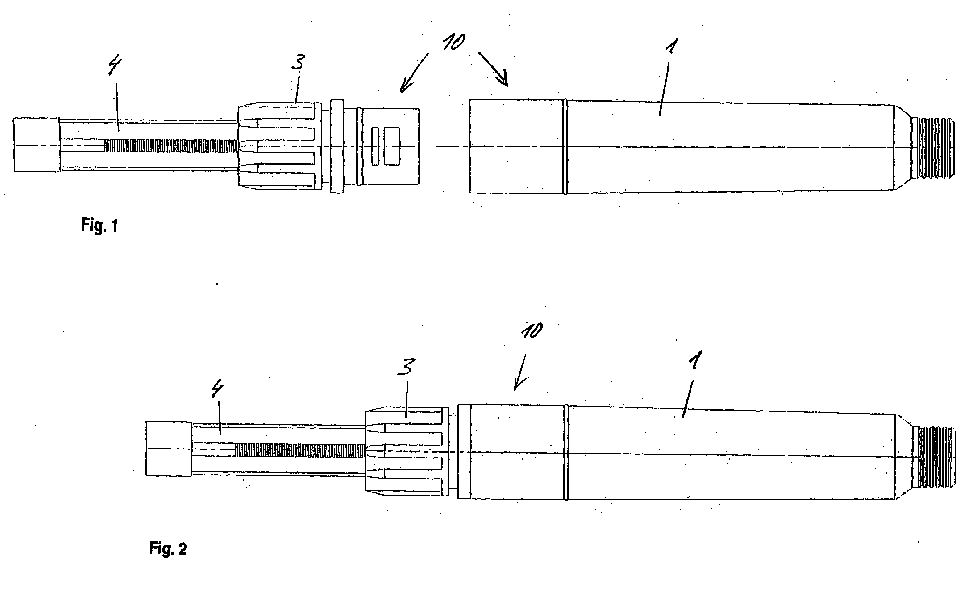

Figur 1 zeigt in einer Ansicht ein Reservoirteil 1 und einen Mechanikhalter 3, die miteinander verbunden werden, um das in Figur 2 dargestellte Reservoirmodul 10 zu bilden.

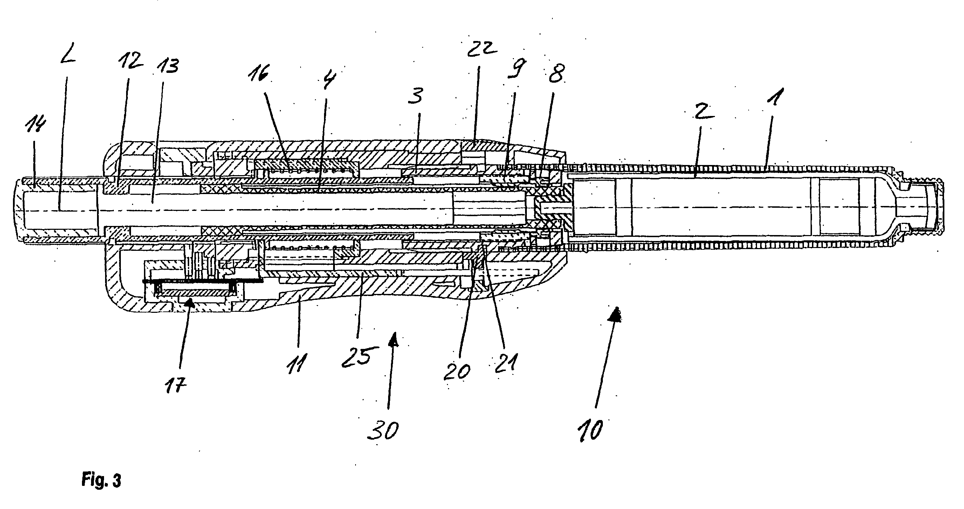

In den Figuren 1 und 2 ist ferner eine Kolbenstange 4 erkennbar, die an einem von dem Reservoirteil 1 abgewandten Ende des Mechanikhalters 3 in den Mechanikhalter 3 hineinragt und von dem Mechanikhalter 3 in eine in Längsachse L der Kolbenstange 4 weisende Vorschubrichtung auf ein von dem Mechanikhalter 3 abgewandtes, vorderes Ende des Reservoirteils 1 zu verschiebbar gelagert wird. Das Reservoirteil 1 ist im Wesentlichen ein im Querschnitt kreisförmiger Hohlzylinder, der an seinem vorderen Ende einen Verbindungsbereich für eine Verbindung mit einem Nadelhalter für eine Injektionsnadel aufweist. Das Reservoirteil 1 dient der Aufnahme eines Reservoirbehältnisses, das im Ausführungsbeispiel von einer Ampulle 2 gebildet wird, die in dem Längsschnitt der Figur 3 erkennbar ist. Ein Auslass an dem vorderen Ende der Ampulle 2 wird von einer Membran fluiddicht verschlossen. Bei der Befestigung des Nadelhalters an dem vorderen Ende des Reservoirteils 1 durchsticht ein rückwärtiger Teil der Injektionsnadel die Membran, so dass eine Fluidverbindung zwischen der Spitze der hohlen Injektionsnadel und dem Reservoir 2 hergestellt ist.

Figur 3 zeigt das Injektionsgerät in seiner Gesamtheit in einem Längsschnitt. In der Ampulle 2 ist ein Kolben in die Vorschubrichtung auf den am vorderen Ende der Ampulle 2 gebildeten Auslass zu verschiebbar aufgenommen. Durch die Verschiebung des Kolbens in Vorschubrichtung wird Produkt aus der Ampulle 2 verdrängt und durch den Auslass und die Injektionsnadel hindurch ausgeschüttet.

Den Vorschub des Kolbens bewirkt die Kolbenstange 4, die mit ihrem vorderen Ende gegen den Kolben drückt und dadurch bei ihrem eigenen Vorschub den Kolben in die Vorschubrichtung bewegt. Die Kolbenstange 4 wird von dem Mechanikhalter 3 so gehalten, dass sie nach Überwindung eines gewissen Widerstands in die Vorschubrichtung, nicht jedoch entgegen der Vorschubrichtung bewegt werden kann. Die Rückwärtsbewegung der Kolbenstange 4 entgegen der Vorschubrichtung wird von einer Blockiereinrichtung 8 verhindert. Die Blockiereinrichtung 8 wird von dem Mechanikhalter 3 axial fixiert, d.h. sie ist in dem Mechanikhalter 3 so gehalten, dass sie in und gegen die Vorschubrichtung nicht bewegbar ist. Allerdings wird sie von dem Mechanikhalter 3 um die Längsachse L drehbar gelagert. Die Blockiereinrichtung 8 erzeugt auch den Widerstand, der für die Vorwärtsbewegung überwunden werden muss.

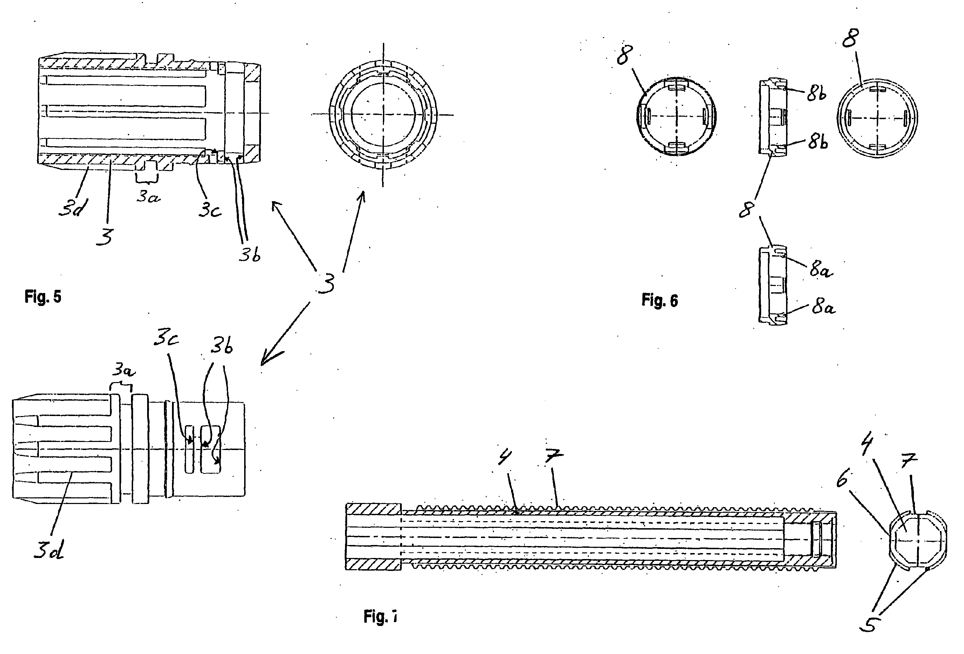

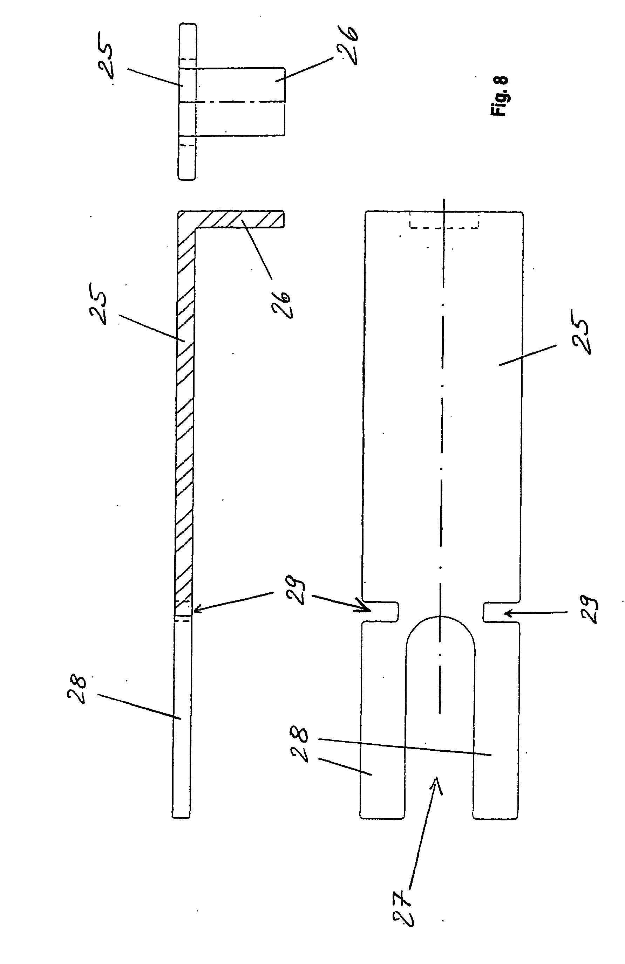

Die Blockiereinrichtung 8 ist in Figur 6 alleine dargestellt. Sie wird von einem einteiligen Ringelement gebildet, das an dem Mechanikhalter 3 zwischen zwei einander zugewandt beabstandeten Schultern 3b, die von einem Innenmantel des Mechanikhaltes 3 radial nach innen vorstehen, um die Längsachse L drehbar anliegt. Die Schultern 3b bilden eine Fixiereinrichtung für die axiale Fixierung der Blockiereinrichtung 8. Die Lagerung der Blockiereinrichtung 8 in dem Mechanikhalter 3 ist am besten aus der Darstellung des Mechanikhalters 3 in Figur 5 ersichtlich.

In dem Mechanikhalter 3 ist ferner ein Dosiseinstellglied 9 aufgenommen. Das Dosiseinstellglied 9 ist als Gewindemutter ausgebildet und steht mit einem Außengewinde der Kolbenstange 4 in einem Gewindeeingriff. Das Dosiseinstellglied 9 wird von dem Mechanikhalter 3 verdrehgesichert, aber in und gegen die Vorschubrichtung axial linear bewegbar geführt. Die Kolbenstange 4 und das Dosiseinstellglied 9 bilden einen Spindeltrieb, um die zu verabreichende Produktdosis auszuwählen.

Der Ampullenhalter 1 und der Mechanikhalter 3 sind verdreh- und verschiebegesichert miteinander verbunden und bilden zusammen das Reservoirmodul 10 des Injektionsgeräts, wobei dieses Reservoirmodul 10 die von dem Mechanikhalter 3 mittels der Blockiereinrichtung 8 gehaltene Kolbenstange 4 und das Dosiseinstellglied 9 umfasst. Der Ampullenhalter 1 und der Mechanikhalter 3 bilden zusammen einen vorderen Gehäuseabschnitt des Injektionsgeräts. Mit diesem vorderen Gehäuseabschnitt 1, 3 ist ein hinterer Gehäuseabschnitt 11 formschlüssig verbunden. Der hintere Gehäuseabschnitt 11 bildet den Träger eines Dosier- und Betätigungselements 12 und zusammen mit demselben sowie Teilen einer Verriegelungseinrichtung und weiteren Teilen ein Dosier- und Betätigungsmodul 30 des Injektionsgeräts.

Eine Dosier- und Betätigungsvorrichtung umfasst mit Ausnahme des Dosiseinstellglieds 9, der Kolbenstange 4 und der Blockiereinrichtung 8 die anderen Komponenten für die Auswahl der Produktdosis und die Betätigung des Injektionsgeräts. Insbesondere umfasst sie das Dosier- und Betätigungselement 12. Ferner umfasst die Dosier- und Betätigungsvorrichtung eine Zähl- und Anzeigeeinrichtung 17 zum Zählen und optischen Anzeigen der ausgewählten Produktdosis. Nicht zuletzt die Zähl- und Anzeigeeinrichtung 17 macht das Dosier- und Betätigungsmodul 30 zu einem hochwertigen und daher hochpreisigen Teil des Injektionsgeräts. Während das vergleichsweise preiswerte Reservoirmodul 10 als Einwegmodul konzipiert ist, ist das Dosier- und Betätigungsmodul 30 für die mehrmalige Verwendung mit immer wieder neuen Reservoirmodulen 10 bestimmt.

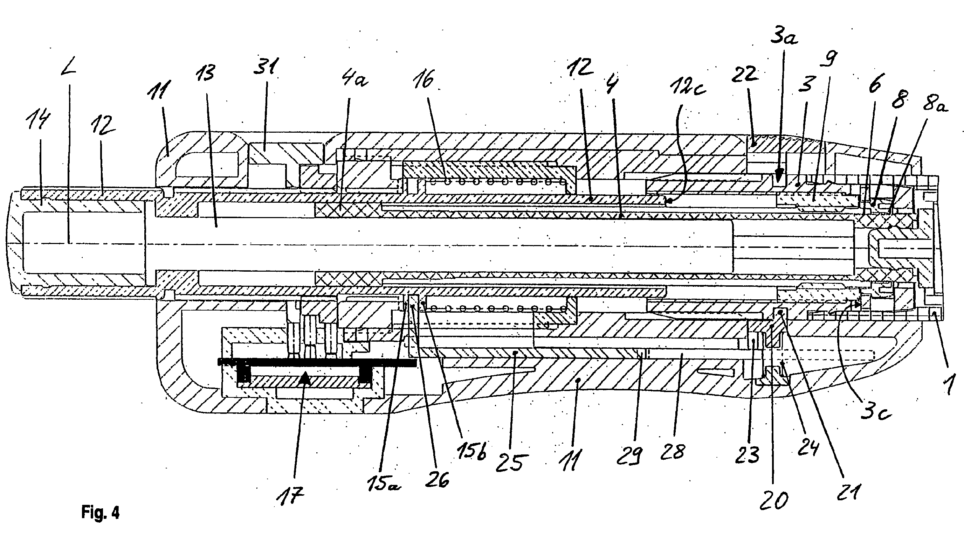

Für die Auswahl der Produktdosis, d.h. für die Dosierung, wird das Dosier- und Betätigungselement 12 um die Längsachse L drehbar und ferner in und gegen die Vorschubrichtung entlang der Längsachse L geradverschiebbar von dem hinteren Gehäuseabschnitt 11 gelagert. Das Dosier- und Betätigungselement 12 ist hohlzylindrisch und umgibt mit einem vorderen Abschnitt die Kolbenstange 4. Ein hinterer Abschnitt des Dosier- und Betätigungselements 12 ragt über ein hinteres Ende des Gehäuseabschnitts 11 hinaus. In das Dosier- und Betätigungselement 12 ist von hinten ein stangenförmiger Dosiermitnehmer 13 bis gegen eine radial nach innen vorragende Schulter des Dosier- und Betätigungselements 12 eingeschoben. Ferner ist an dem hinteren Ende ein Verschluss 14 bis gegen den Dosiermitnehmer 13 in das Dosier- und Betätigungselement 12 eingeschoben. Der Dosiermitnehmer 13 wird zwischen der radial vorragenden Schulter des Dosier- und Betätigungselements 12 und dem Verschluss 14 relativ zu dem Dosier- und Betätigungselement 12 axial fixiert. Der Dosiermitnehmer 13 ist mit dem Dosier- und Betätigungselement 12 außerdem verdrehgesichert verbunden. Der Dosiermitnehmer 13 ragt zum Zwecke der Dosierung von hinten in die hohle Kolbenstange 4 hinein. Die Kolbenstange 4 weist einen Verbindungabschnitt 4a auf (Fig. 4), der mit dem Dosiermitnehmer 13 in solch einem Eingriff ist, dass die Kolbenstande 4 und der Dosiermitnehmer 13 und mit diesem auch das Dosier- und Betätigungselement 12 relativ zueinander um die gemeinsame Längsachse L nicht verdrehbar, aber entlang der Längsachse L in und gegen die Vorschubrichtung relativ zueinander bewegbar sind. Hierfür ist der Verbindungsabschnitt 4a als Linearführung für den Dosiermitnehmer 13 ausgebildet.

Eine Rückstelleinrichtung 16 spannt das Dosier- und Betätigungselement 12 elastisch gegen die Vorschubrichtung in die in den Figuren 3 und 4 dargestellte Ausgangsstellung. In der Ausgangsstellung kann durch Drehung des Dosier- und Betätigungselements 12 um die Längsachse L die Dosierung vorgenommen werden. Anschließend kann aus der Ausgangsstellung heraus durch Axialverschiebung des Dosier- und Betätigungselements 12 die Ausschüttung der ausgewählten Produktdosis bewirkt werden. Die Rückstelleinrichtung 16 wird von einer als Druckfeder wirkenden Spiralfeder gebildet, die in einem Ringspalt um das Dosier- und Betätigungselement 12 aufgenommen ist und zwischen einer radial nach innen ragenden Schulter des Gehäuseabschnitts 11 und einer gegenüberliegend zugewandt radial nach außen ragenden Schulter des Dosier- und Betätigungselements 12 axial abgestützt ist.

Die Blockiereinrichtung 8 erfüllt eine Doppelfunktion. Zum einen stellt sie mit ihren Sperrelementen 8a sicher, dass die Kolbenstange 4 relativ zu dem Mechanikhalter 3 und damit insbesondere relativ zu dem in der Ampulle 2 aufgenommenen Kolben nicht entgegen der Vorschubrichtung zurückbewegt werden kann. In ihrer Doppelfunktion verhindert die Blockiereinrichtung 8 ferner als Bremse eine Vorwärtsbewegung der Kolbenstange 4 bei dem Dosiervorgang, bei dem das Dosiseinstellglied 9 entgegen der Vorschubrichtung axial auf das Dosier- und Betätigungselement 12 zu bewegt wird.

In der in den Figuren 3 und 4 dargestellten Ausgangsstellung vor einer Dosierung liegt das Dosiseinstellglied 9 in Vorschubrichtung auf Anschlag gegen einen von dem Mechanikhalter 3 gebildeten Ausschüttanschlag 3c (Figur 5). Die Kolbenstange 4 steht in einem permanenten Berührungskontakt mit dem Kolben. Zum Zwecke der Dosierung wird das Dosiseinstellglied 9 durch den Gewindeeingriff mit der Kolbenstange 4 und die Linearführung durch den Mechanikhalter 3 von dem Ausschüttanschlag 3c weg auf das Dosier- und Betätigungselement 12 zu bewegt. Hierdurch wird ein lichter Abstand zwischen einer rückwärtigen Anschlagfläche des Dosiseinstellglieds 9 und einer vorderen Anschlagfläche des Dosier- und Betätigungselements 12 verringert, andererseits jedoch der lichte Abstand zwischen einer vorderen Anschlagfläche des Dosiseinstellglieds 9 und dem Ausschüttanschlag 3c vergrößert. Der letztgenannte Abstand zwischen dem Ausschüttanschlag 3c und dem Dosiseinstellglied 9 ist die Weglänge, um die im Zuge der Ausschüttbewegung des Dosier- und Betätigungselements 12 das Dosiseinstellglied 9 und wegen des Gewindeeingriffs auch die Kolbenstange 4 in die Vorschubrichtung bewegt werden. Der Ausschüttanschlag 3c bildet einen vorderen Translationsanschlag. Bei der Ausschüttbewegung drückt die Kolbenstange 4 mit ihrem vorderen Ende, das von einem mit der Kolbenstange 4 in und gegen die Vorschubrichtung nicht bewegbar verbundenen Stempelkörper gebildet wird, gegen den Kolben und drückt den Kolben in die Vorschubrichtung auf den Auslass der Ampulle 2 zu vor. Die Längsachse L bildet die Rotations- und Translationsachse der Bewegungen, die zum Zwecke der Dosierung und Produktausschüttung ausgeführt werden.

Der Abstand, den das Dosiseinstellglied 9 und das Dosier- und Betätigungselement 12 vor dem Dosiervorgang zwischen sich aufweisen, wenn das Dosiseinstellglied 9 auf Anschlag gegen den Ausschüttanschlag 3c liegt, entspricht der maximalen Produktdosis, die ausgewählt und im Zuge einer Ausschüttung ausgeschüttet werden kann. Die Hubbewegung des Dosier- und Betätigungselements 12 ist bei jeder Ausschüttung gleich lang. Es wird durch die Dosierung lediglich der Abstand des Dosiseinstellglieds 9 von dem Ausschüttanschlag 3c und damit die im Zuge der Ausschüttung von dem Dosier- und Betätigungselement 12 und dem Dosiseinstellglied 9 gemeinsam zurücklegbare Weglänge eingestellt.

Die Bremsfunktion der Blockiereinrichtung 8 und der hierfür zwischen der Kolbenstange 4 und der Blockiereinrichtung 8 bestehende Bremseingriff werden aus der Zusammenschau der Figuren 6 und 7 deutlich. Zum einen weist die Blockiereinrichtung 8 für den Bremseingriff zwei Bremselemente 8b auf, die, wie bereits die Sperrelemente 8a, je von einem elastisch nachgebenden Schnapper gebildet werden. Die Blockiereinrichtung 8 wird im Ausführungsbeispiel von einem einzigen Ringelement gebildet, von dem an einer Stirnseite vier elastische Schnapper axial abragen. Die Schnapper sind gleichmäßig über den Umfang des Ringelements verteilt angeordnet. Zwei einander gegenüberliegende Schnapper bilden die Sperrelemente 8a und die zwei weiteren, einander ebenfalls gegenüberliegend angeordneten Schnapper bilden die Bremselemente 8b.

Die Kolbenstange 4 weist dementsprechend zwei am Außenmantel an gegenüberliegenden Seiten ausgebildete und in Längsrichtung der Kolbenstange 4 sich erstreckende Rückzugsperreinrichtungen 6 und zwei an ebenfalls einander gegenüberliegenden Seiten in Längsrichtung der Kolbenstange 4 sich erstreckende Vorschubbremseinrichtungen 7 auf. Das Gewinde der Kolbenstange 4 für den Gewindeeingriff mit dem Dosiseinstellglied 9 wird von vier verbleibenden Gewindeabschnitten 5 gebildet, die sich über nahezu die gesamte Länge der Kolbenstange 4 erstrecken. Die Rückzugsperreinrichtungen 6 und die Vorschubbremseinrichtungen 7 werden je von einer Zahnreihe gebildet. Während die Zähne der Rückzugsperreinrichtungen 6 jedoch als in Vorschubrichtung gepfeilte Sägezähne mit nach hinten weisenden, quer zur Vorschubrichtung sich erstreckenden Sperrflächen gebildet sind, weisen die beiden Zahnreihen, die die Vorschubbremseinrichtungen 7 bilden, nach vorne weisende Sperrflächen mit einer vergleichbaren Sperrwirkung nicht auf. Die Zähne der Vorschubbremseinrichtungen 7 weisen je ein im Vergleich zu den Rückzugssperreinrichtungen 6 weicheres Zahnprofil auf. Der Bremseingriff der Blockiereinrichtung 8 mit den Vorschubbremseinrichtungen 7 der Kolbenstange 4 soll eine Vorschubbewegung der Kolbenstange 4 nämlich nicht verhindern, sondern nur erschweren, um sicherzustellen, dass die Kolbenstange 4 nicht bei der Dosierung in Vorschubrichtung bewegt wird. Die Zähne der Vorschubbremseinrichtungen 7 sind an ihren Vorderseiten und die Bremselemente 8b sind an ihren Rückseiten, die die Vorderseiten der Zähne der Vorschubbremseinrichtung 7 berühren, so geformt, dass zur Überwindung des Bremseingriffs eine Schwellkraft überwunden werden muss, die bei der Dosierung nicht erreicht wird. Diese Schwellkraft ist größer als die Kraft, die erforderlich ist, um die Zähne der Rückzugssperreinrichtungen 6 über die Sperrelemente 8a in Vorschubrichtung zu bewegen. Vorzugsweise ist die Schwellkraft wenigstens doppelt so groß wie die anfängliche Reibkraft zwischen den Rückzugssperreinrichtungen 6 und den Sperrelementen 8a. Die Reibkraft zwischen letzteren vergrößert sich auch erst im Verlaufe der Vorschubbewegung allmählich zwischen zwei aufeinanderfolgenden Sperreingriffen. Die Schwellkraft des Bremseingriffs muss hingegen in jedem Sperreingriff unmittelbar am Beginn der Vorschubbewegung von einem Sperreingriff in den nächst folgenden aufgebracht werden. Allerdings soll die Schwellkraft nicht so groß sein, dass sie vom Verwender bei der Ausschüttung als störend empfunden wird.

Eine unerwünschte Vorschubbewegung der Kolbenstange als Reaktion auf die Bewegung des Dosiseinstellglieds 9 bei der Dosisauswahl kann grundsätzlich auch allein durch den Sperreingriff der Blockiereinrichtung 8 bewirkt werden. Allerdings wird solch eine Bewegung wegen des Bremseingriffs sicherer verhindert als durch den Sperreingriff allein.

Die Verbindung zwischen dem Reservoirmodul 10 und dem Dosier- und Betätigungsmodul 30 ist formschlüssig. Zum einen besteht zwischen dem Mechanikhalter 3 und dem Gehäuseabschnitt 11 ein Verriegelungseingriff, der eine Relativbewegung in axialer Richtung verhindert. Über den Verriegelungseingriff hinaus sind der vordere Gehäuseabschnitt 1, 3 und der hintere Gehäuseabschnitt 11 unmittelbar aneinander axial linear geführt, um eine Relativdrehung bei dem Verbinden und im verbundenem Zustand zu verhindern. In Figur 5 sind die Axialführungen 3d des Mechanikhalters 3, die mit einem oder mehreren entsprechenden Eingriffselementen des hinteren Gehäuseabschnitts 11 die Linearführung bilden, gut zu erkennen. Die Axialführungen 3d werden von Führungsflächen an Führungsrippen gebildet; sie könnten auch von Führungsflächen in axial sich erstreckenden Vertiefungen gebildet werden. Auf diese Weise werden axiale Führungskanäle erhalten. Die Führungsrippen sind axial verjüngt, so dass in die Führungskanäle führende Einführtrichter für das eine oder die mehreren Eingriffselemente des hinteren Gehäuseabschnitts 11 gebildet werden. Zur noch besseren Zentrierung der Gehäuseabschnitte 1, 3 und 11 zu Beginn des Verbindens sind die Führungsrippen auch noch in radialer Richtung verjüngt. Das eine oder die mehreren Eingriffselemente des hinteren Gehäuseabschnitts 11 ist oder sind vorzugsweise wie die Axialabschnitte 3d an der Mantelgegenfläche, d.h. der Innenmantelfläche, des hinteren Gehäuseabschnitts 11 gebildet.

Der Verriegelungseingriff besteht zwischen einem weiblichen, ersten Verriegelungselement 3a des Mechanikhalters 3 (Figur 5) und einem Verriegelungsring 20, der mit dem hinteren Gehäuseabschnitt 11 radial bewegbar, aber axial nicht bewegbar verbunden ist. Der Verriegelungsring 20 bildet ein unmittelbar in das erste Verriegelungselement 3a radial eingreifendes männliches, zweites Verriegelungselement 21. Zwischen dem ersten Verriegelungselement 3a und dem zweiten Verriegelungselement 21 besteht eine Schloss/Riegel-Verbindung, die axiale Relativbewegungen zwischen dem Reservoirmodul 10 und dem Dosier- und Betätigungsmodul 30 verhindert.

Die Figuren 3 und 4 zeigen das Verriegelungselement 21 im Verriegelungseingriff mit dem Verriegelungselement 3a. Das Verriegelungselement 3a wird von einem Ringsteg und einer Nut gebildet, die an dem Außenmantel des Mechanikhalters 3 umlaufen. Der Ringsteg bildet eine hintere Seitenwand der Nut. Das zweite Verriegelungselement 21 wird von einem Nocken gebildet, der von dem Innenmantel des Verriegelungsrings 20 radial einwärts vorragt und im Verriegelungseingriff von einer Rückstelleinrichtung 24 radial einwärts über eine Innenmantelfläche des hinteren Gehäuseabschnitts 11 vorstehend in das aufnehmende Verriegelungselement 3a hineingedrückt wird. Der Verriegelungsring 20 ist in seiner Gesamtheit in Radialrichtung mittels der Rückstelleinrichtung 24 an einer von dem Gehäuseabschnitt 11 gebildeten Innenmantelfläche abgestützt, so dass die Rückstelleinrichtung 24 etwa in radialer Verlängerung des Verriegelungselements 21 gegen den Außenmantel des Verriegelungsrings 20 drückt. Der Verriegelungsring 20 umgibt den Mechanikhalter 3 und ist in seiner Gesamtheit gegen die rückstellende Kraft der Rückstelleinrichtung 24 radial hin und her bewegbar, so dass das zweite Verriegelungselement 21 in und aus dem Verriegelungseingriff mit dem ersten Verriegelungselement 3a bringbar ist. Der hintere Gehäuseabschnitt 11 bildet eine enge Gleitführung für die Radialbewegung des Verriegelungsrings 20. An seiner dem Verriegelungselement 21 radial gegenüberliegenden Seite bildet der Verriegelungsring 20 einen Entriegelungsknopf 22 für den Verwender. Zur radialen Führung der als Druckfeder ausgebildeten Rückstelleinrichtung 24 ragt von der dem Verriegelungselement 21 abgewandten Außenmantelfläche des Verriegelungsrings 20 ein Führungsnocken radial ab.

In Umfangsrichtung zu beiden Seiten dieses Führungsnockens und axial hinter dem Führungsnocken ragen von der Außenmantelfläche des Verriegelungsrings 20 ferner zwei Sperrnocken 23 ab, die in Radialrichtung nach außen gegen eine Verriegelungssperre 25 drücken. Aufgrund der Anlage der Sperrnocken 23 gegen die Verriegelungssperre 25 wird eine Radialbewegung des Verriegelungselements 21 verhindert, die zu einem Lösen des Verriegelungseingriffs führen könnte. Der zwischen den Verriegelungselementen 3a und 21 bestehende Verriegelungseingriff wird somit durch die Verriegelungssperre 25 gesichert. Diese Sicherung besteht in jeder Verschiebeposition des Dosier- und Betätigungselements 12 mit Ausnahme einer Freigabestellung, die das Dosier- und Betätigungselement 12 am Ende seiner Ausschüttbewegung einnimmt. Die Freigabestellung fällt daher mit der vordersten Verschiebeposition zusammen, die das Dosier- und Betätigungselement 12 dann einnimmt, wenn es im Zuge seiner Ausschüttbewegung an das Dosiseinstellglied 9 und das Dosiseinstellglied 9 seinerseits gegen den Ausschüttanschlag 3c des Mechanikhalters 3 anstößt. Solange das Dosier- und Betätigungsmodul 30 mit dem Reservoirmodul noch nicht verbunden ist, wird ein mechanischer Anschlag für das Dosier- und Betätigungselement 12 von einem Anschlagelement 31 der Dosier- und Betätigungsvorrichtung gebildet. Im Ausführungsbeispiel bildet ein Resethalterring, der einem Reset der Anzeige 17 dient, das Anschlagelement 31. Der Anschlag des Dosier- und Betätigungselement 12 gegen dieses Anschlagelement 31 definiert die Freigabestellung des Dosier- und Betätigungselements 12 in diesem Falle, wobei die durch das Anschlagelement 31 definierte Freigabestellung derjenigen entspricht, die durch das Anstoßen des Dosiseinstellglieds 9 an dem Ausschüttanschlag 3c definiert wird.

Figur 8 zeigt die Verriegelungssperre 25. Sie wird im Ausführungsbeispiel einstückig von einem Sperrschieber gebildet. Die Verriegelungssperre 25 weist einen plattenförmigen Hauptkörper auf, der sich im montierten Zustand, wie beispielsweise in Figur 4 dargestellt, axial erstreckt. An einem Ende ragt von dem Hauptkörper ein Steg 26 rechtwinklig ab. Im montierten Zustand erstreckt sich der Steg 26 radial bis gegen das Dosier- und Betätigungselement 12. Der Steg 26 dient der Befestigung der Verriegelungssperre 25 an dem Dosier- und Betätigungselement 12, das hierfür zwei axial beabstandet an einer Außenmantelfläche gebildete Ringstege aufweist, die Mitnehmer 15a und 15b bilden. Der vordere Mitnehmer 15b bildet gleichzeitig die Stützschulter für die Rückstelleinrichtung 16. In den zwischen den Mitnehmern 15a und 15b gebildeten Ringraum ragt die Verriegelungssperre 25 mit ihrem Steg 26 ein und wird von den beiden Mitnehmern 15a und 15b axial beidseits eng eingefasst.

An einem von dem Steg 26 abgewandten vorderen Ende ist der Hauptkörper der Verriegelungssperre 25 mit einer Axialausnehmung 27 versehen, die zu dem vorderen Ende der Verriegelungssperre 25 hin offen ist. Auf diese Weise werden beidseits der Ausnehmung 27 axial sich erstreckende Sperrzungen 28 gebildet. Die beiden Sperrnocken 23 des Verriegelungsrings 20 sind so angeordnet, dass je einer dieser Sperrnocken 23 gegen eine der Sperrzungen 28 drückt, solange das Dosier- und Betätigungselement 12 nicht die Freigabestellung einnimmt. Durch die axiale Ausnehmung 27 hindurch erstreckt sich bei der Axialbewegung der Verriegelungssperre 25 die Rückstelleinrichtung 24 für das Verriegelungselement 21.

In dem Hauptkörper der Verriegelungssperre 25 sind ferner Einrückausnehmungen 29 gebildet, die die Freigabestellung des Dosier- und Betätigungselements 12 definieren. Pro Sperrnocken 23 ist je eine Einrückausnehmung 29 vorgesehen. Die Position der Einrückausnehmungen 29 ist so gewählt, dass sie mit den Sperrnocken 23 erst dann in Überdeckung gelangen und dadurch ein Einfahren der Sperrnocken 23 gestatten, wenn das Dosier- und Betätigungselement 12 bis in seine Freigabestellung vorbewegt worden ist.

Es ist klar, dass bei der im Ausführungsbeispiel speziell gewählten Anordnung auch ein einziger Sperrnocken 23 vorgesehen sein könnte und die Verriegelungssperre 25 dementsprechend auch nur eine einzige Einrückausnehmung 29 und gegebenenfalls auch nur eine Sperrzunge 28 aufweisen könnte. Grundsätzlich könnte die Verriegelungssperre ferner einstückig mit dem Dosier- und Betätigungselement 12 gefertigt sein. Die Ausbildung als separates Teil bietet jedoch Vorteile hinsichtlich der Fertigung, der Montage und dem Zusammenwirken des Dosier- und Betätigungselements 12 mit der Kolbenstange 4. In Bezug auf die Einbaulage der Verriegelungssperre 25 ist noch darauf hinzuweisen, dass die Verriegelungssperre 25 an ihrer von dem Verriegelungselement 21 abgewandten Außenseite an einer Innenmantelfläche des Gehäuses 11 abgestützt ist. Auf diese Weise wird die Stabilität der Sicherung für den Verriegelungseingriff erhöht. Vorzugsweise bildet das Gehäuse 11 eine Axialführung für die Verriegelungssperre 25.

Im Folgenden wird die Funktionsweise des Injektionsgeräts beschrieben, wobei angenommen sei, dass ein neues Reservoirmodul 10 und ein bereits wenigstens einmal verwendetes Dosier- und Betätigungsmodul 30 zusammengebaut und anschließend eine erste Produktausschüttung vorgenommen werden.

Das Dosier- und Betätigungsmodul 30 und das neue Reservoirmodul 10 werden axial zueinander ausgerichtet, so dass ihre beiden Längsachsen miteinander fluchten. Anschließend wird das Reservoirmodul 10 mit seinem rückwärtigen Ende in das nach vorne offene Gehäuse 11 des Dosier- und Betätigungsmoduls 30 eingeführt.

Hierbei zentrieren sich der Gehäuseabschnitt 1, 3 und der Gehäuseabschnitt 11 an den verjüngten Enden der Führungsrippen 3d des Mechanikhalters 3. Während des Aufschiebens werden die beiden Gehäuseabschnitte in einer durch die Linearführung vorgegebenen Drehwinkelposition axial aneinander lineargeführt, bis die Gehäuseabschnitte 1, 3 und 11 eine Verbindungsendposition einnehmen, in der der Verriegelungseingriff der Verriegelungselemente 3a und 21 hergestellt werden kann oder von selbst sich einstellt.

Das Dosier- und Betätigungselement 12 ist in vorgegebenen Drehwinkelpositionen relativ zu dem hinteren Gehäuseabschnitt 11 gerastet. Die Linearführung der Gehäuseabschnitte 1, 3 und 11 und die Drehwinkelrastposition des Dosier- und Betätigungselements 12 sind so aufeinander abgestimmt, dass der verdrehgesicherte Eingriff des Dosier- und Betätigungselements 12 mit der Kolbenstange 4 in jeder Rastposition des Dosier- und Betätigungselements 12 und jeder Drehwinkelposition, in der die Gehäuseabschnitte 1, 3 und 11 aneinander lineargeführt sind, hergestellt wird.

Falls das Dosier- und Betätigungselement 12 sich in einer Axialposition relativ zu dem Gehäuseabschnitt 11 befindet, die hinter der Freigabestellung liegt, wird das Verriegelungselement 21 von der Verriegelungssperre 25 in seiner radial innersten Stellung gehalten. In dieser Stellung des Verriegelungselements 21 können das Dosier- und Betätigungsmodul 30 und das Reservoirmodul 10 nicht bis in die Verbindungsendstellung aufeinandergeschoben und deshalb auch nicht miteinander verbunden werden, da der am Außenmantel des Mechanikhalters 3 gebildete Ringsteg, der das erste Verriegelungselement 3a mitbildet, vorher gegen das zweite Verriegelungselement 21 auf Anschlag zu liegen kommt.

Der Ringsteg kann zu einem in tangentialer Richtung kurzen Radialvorsprung reduziert werden, wenn dafür gesorgt ist, dass die Gehäuseabschnitte 1, 3 und 11 nur in der Drehwinkellage zusammengebaut werden können, in der solch ein Vorsprung und das zweite Verriegelungselement 21 in einer axialen Flucht zu liegen kommen. Der Ringsteg oder Radialvorsprung könnte das erste Verriegelungselement 3a auch alleine bilden, da es die wesentliche Funktion des ersten Verriegelungselements 3a ist, die Herstellung der Verbindung zwischen dem Reservoirmodul 10 und dem Dosier- und Betätigungsmodul 30 nur zuzulassen, wenn das Dosier- und Betätigungselement 12 seine Freigabestellung einnimmt. Ist diese Bedingung erfüllt, so würde das Dosier- und Betätigungselement 12 bei der Herstellung der Verbindung zwischen dem Reservoirmodul 10 und dem Dosier- und Betätigungsmodul 30 sicherstellen, dass das Dosiseinstellglied 9 sich in seiner Dosiernullstellung befindet, in der es an dem Ausschüttanschlag 3c des Mechanikhalters 3 anstößt.

Um den die vorstehend erläuterte Bedingung erfüllenden Zustand herzustellen, drückt der Verwender das Dosier- und Betätigungselement 12 axial relativ zu dem hinteren Gehäuseabschnitt 11 bis in die Freigabestellung vor. In dieser Relativstellung zwischen Gehäuseabschnitt 11 und Dosier- und Betätigungselement 12 können die Sperrnocken 23 in die Einrückaufnahmen 29 der Verriegelungssperre 25 bewegt werden. Der Verwender drückt daher nicht nur das Dosier- und Betätigungselement 12 bis wenigstens in die Freigabestellung vor, sondern gleichzeitig auch mittels des Entriegelungsknopfs 22 das erste Verriegelungselement 20 aus dem Verriegelungseingriff. Das Reservoirmodul 10 kann nun axial über den Ringsteg des ersten Verriegelungselements 3a bewegt und weiter in den hinteren Gehäuseabschnitt 11 eingeführt werden. Der Verwender kann den Entriegelungsknopf 22 loslassen. Sobald das erste Verriegelungselement 21 mit dem zweiten Verriegelungselement 3a in Überdeckung gelangt, schnappt es aufgrund der Kraft der Rückstelleinrichtung 24 in das aufnehmende Verriegelungselement 3a ein, so dass der Verriegelungseingriff hergestellt ist. Das Reservoirmodul 10 und das Dosier- und Betätigungsmodul 30 sind nun in Bezug auf die Stellung des Dosiseinstellglieds 9 und der Kolbenstange 4 in definierter Weise miteinander verbunden. Falls das Dosiseinstellglied 9 vor Herstellung des Verriegelungseingriffs noch einen lichten Abstand von dem Ausschüttanschlag 3c aufgewiesen hat, ist dieser Abstand aufgrund der zum Herstellen der Verbindung erforderlichen Einwirkung des Dosier- und Betätigungselements 12 beseitigt. Eine damit einhergehende Produktausschüttung kann hingenommen werden und kann zum Zwecke des Primens der Injektionsnadel sogar erwünscht sein. Die Zähl- und Anzeigeeinrichtung 17 wird vorzugsweise dabei genullt.

In dem derart herbeigeführten, definierten Ausgangszustand kann der Verwender die Dosierung vornehmen. Die Dosierung erfolgt durch Drehung des Dosier- und Betätigungselements 12 um die Längsachse L und relativ zu dem Gehäuseabschnitt 11. Da der Dosiermitnehmer 13 mit dem Dosier- und Betätigungselement 12 verdrehgesichert verbunden ist und seinerseits verdrehgesichert in die Kolbenstange 4 eingreift, nimmt das Dosier- und Betätigungselement 12 bei der rotatorischen Dosierbewegung die Kolbenstange 4 mit. Aufgrund des Gewindeeingriffs zwischen der Kolbenstange 4 und dem Dosiseinstellglied 9 und der Linearführung des Dosiseinstellglieds 9 durch den Mechanikhalter 3 führt das Dosiseinstellglied 9 eine von der Gewindesteigung des gegenseitigen Gewindeeingriffs vorgegebene, axiale translatorische Dosierbewegung in Richtung auf das Dosier- und Betätigungselement 12 aus. Das Dosier- und Betätigungselement 12 bildet einen hinteren Translationsanschlag 12c, der die translatorische Dosierbewegung des Dosiseinstellglieds 9 begrenzt und dadurch den maximal einstellbaren Ausschütthub definiert.

Die Zähl- und Anzeigeeinrichtung 17 zählt die der Drehwinkelstellung des Dosier- und Betätigungselements 12 entsprechenden Dosiseinheiten und zeigt sie optisch an.

Nach Auswahl der gewünschten Produktdosis ist der Dosiervorgang beendet. Die Ausschüttung der gewählten Produktdosis wird mittels der in Vorschubrichtung des Kolbens weisenden Ausschüttbewegung des Dosier- und Betätigungselements 12 bewirkt. Im Zuge seiner Ausschüttbewegung stößt das Dosier- und Betätigungselement 12 gegen das Dosiseinstellglied 9 und nimmt es mit. Indem das Dosiseinstellglied 9 im Zuge der Ausschüttbewegung gegen den Ausschüttanschlag 3c des Mechanikhalters 3 stößt, werden die Ausschüttbewegungen des Dosier- und Betätigungselements 12 und die Produktausschüttung beendet. Nach dem Loslassen des Dosier- und Betätigungselements 12 vorzugsweise wird dieses von dem Rückstelleinrichtung 16 entgegen der Vorschubrichtung wieder in eine neue Ausgangsstellung für eine erneute Dosierung und Produktausschüttung bewegt. Die Zähl- und Anzeigeeinrichtung 17 ist mit dem Dosier- und Betätigungselement 12 vorzugsweise so gekoppelt, dass sie mittlerweile wieder auf Null zurückgestellt worden ist. Gegebenenfalls verfügt sie über Mittel zum Zählen und Anzeigen der bereits insgesamt ausgeschütteten Produktmenge und somit der in der Ampulle 2 verbliebenen Produktrestmenge.

Um das Reservoirmodul 10 von dem Dosier- und Betätigungsmodul 30 zu lösen, wird das Dosier- und Betätigungselement 12 bis in die Freigabestellung, d.h. bis auf Anschlag gegen das Dosiseinstellglied 9, vorbewegt. In dieser Stellung kann der Verwender durch Druck auf den Entriegelungsknopf 22 den Verriegelungseingriff wieder lösen und das Reservoirmodul 10 von dem Dosier- und Betätigungsmodul 30 trennen.

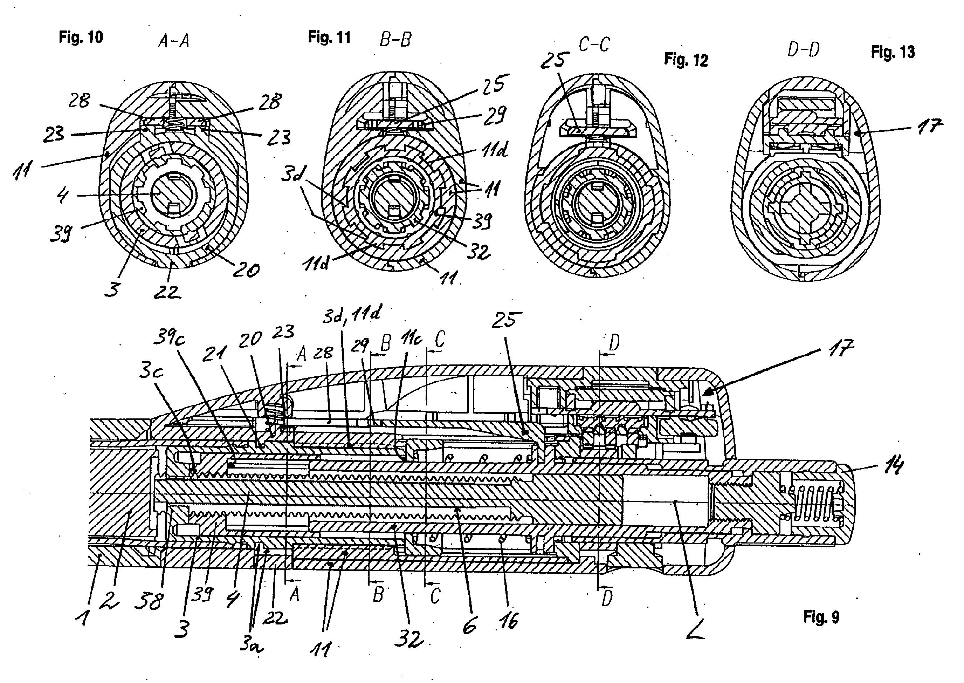

Die Figuren 9 bis 13 zeigen in einem Längsschnitt und vier Querschnitten ein zweites Ausführungsbeispiel eines Injektionsgeräts. Das injektionsgerät des zweiten Ausführungsbeispiels gleicht demjenigen des ersten Ausführungsbeispiels in Bezug auf die Verriegelung und die Verriegelungssperre 25 derart, dass auf die diesbezügliche Beschreibung des ersten Ausführungsbeispiels verwiesen wird. Insbesondere ist die Verriegelungssperre 25 des zweiten Ausführungsbeispiels in Bezug auf alle funktionalen Details mit derjenigen des ersten Ausführungsbeispiels identisch. Das gleiche gilt für die Verriegelungselemente 3a und 21.

Der Verriegelungsring 20 und die Lage der Sperrnocken 23 relativ zu dem Verriegelungselement 21 und relativ zu der Verriegelungssperre 25 in dem Ausgangszustand des Geräts sind besonders gut in den Querschnitten der Figuren 10, 11 und 12 zu erkennen, auf die diesbezüglich auch stellvertretend für das erste Ausführungsbeispiel verwiesen wird.

Das Injektionsgerät des zweiten Ausführungsbeispiels unterscheidet sich von dem ersten Ausführungsbeispiel durch den Eingriff und den Bewegungsablauf der an der Dosierung beteiligten Komponenten. Ferner erfüllt der Mechanikhalter über die Funktionen des Mechanikhalters des ersten Ausführungsbeispiels hinaus insbesondere die Funktion der Positionierung des Dosiseinstellglieds in diskreten Drehwinkelpositionen, die relativ zu dem Mechanikhalter zum Zwecke der Dosierung veränderbar sind. Die Blockiereinrichtung des zweiten Ausführungsbeispiels ist hingegen einfacher ausgeführt als die des ersten Ausführungsbeispiels. Im Folgenden werden in erster Linie nur die Unterschiede im Vergleich zum ersten Ausführungsbeispiel beschrieben, wobei für Komponenten, die ihrer grundsätzlichen Funktion nach den gleichnamigen Komponenten des ersten Ausführungsbeispiels gleichen, aber in Details unterscheiden, 30iger Zahlen mit gleicher Endziffer oder exakt die gleichen Bezugszeichen, wie bei dem ersten Ausführungsbeispiel, verwendet werden. Soweit zum zweiten Ausführungsbeispiel keine Ausführungen gemacht werden, sollen die entsprechenden Ausführungen zum ersten Ausführungsbeispiel gelten.

In dem zweiten Ausführungsbeispiel ist das relativ zu dem hinteren Gehäuseabschnitt 11 axial linear bewegbare und um die Längsachse L verdrehbare Dosier- und Betätigungselement 32 mit dem Dosiseinstellglied 39 verdrehgesichert verbunden. Das Dosier- und Betätigungselement 32 und das Dosiseinstellglied 39 sind relativ zueinander und relativ zu den Gehäuseabschnitten 1, 3 und 11 in und gegen die Vorschubrichtung bewegbar. Die Kolbenstange 4 wird von dem Mechanikhalter 3 verdrehgesichert gehalten. Die mit dem ersten Ausführungsbeispiel funktionsgleiche Rückzugsperreinrichtung 6 verhindert im Zusammenwirken mit Sperrelementen der einstückig an dem Mechanikhalter 3 geformten Blockiereinrichtung 38 eine Bewegung der Kolbenstange 4 entgegen der Vorschubrichtung, lässt eine Bewegung in die Vorschubrichtung jedoch zu. Die Sperrelemente bilden gleichzeitig die Rückzugsperre und die Verdrehsicherung für die Kolbenstange 4. Des Weiteren bildet wie bereits im ersten Ausführungsbeispiel das Dosier- und Betätigungselement 32 eine Gleitführung für die Kolbenstange 4.

Bei der Dosierung führt das Dosier- und Betätigungselement 32 die gleiche rotatorische Dosierbewegung wie das Dosier- und Betätigungselement 12 des ersten Ausführungsbeispiels aus. Allerdings wird aufgrund des verdrehgesicherten Eingriffs das Dosiseinstellglied 39 bei der rotatorischen Dosierbewegung mitgenommen. Der Gewindeeingriff zwischen der Kolbenstange 4 und dem Dosiseinstellglied 39 ist wieder mit demjenigen des ersten Ausführungsbeispiels vergleichbar, so dass ein von dem Dosiseinstellglied 39 gebildeter Anschlag 39c aufgrund der rotatorischen Dosierbewegung und des Gewindeeingriffs mit der Kolbenstange 4 im Zuge der Dosierung entgegen der Vorschubrichtung auf ein vorderes Ende des Dosier- und Betätigungselements 32 zu bewegt wird. Im Gegensatz zum ersten Ausführungsbeispiel vollführt das Dosiseinstellglied 39 somit bei der Dosierung eine rotatorische Dosierbewegung und eine translatorische Dosierbewegung relativ zu dem vorderen Gehäuseabschnitt, während die Kolbenstange 4 stillsteht. Durch die nach Abschluss der Dosierung erfolgende Ausschüttbewegung des Dosier- und Betätigungselements 32 wird die Kolbenstange 4 um die Weglänge vorgeschoben, die dem durch die Dosierung eingestellten lichten Abstand zwischen einer Anschlagfläche des Dosiseinstellglieds 39 und dem Ausschüttanschlag 3c des Mechanikhalters 3 entspricht.

Die translatorische Dosierbewegung des Dosiseinstellglieds 39 wird gegen die Vorschubrichtung von einem hinteren Translationsanschlag 11 c begrenzt, den der hintere Gehäuseabschnitt 11 selbst unmittelbar bildet. Auch bei dem zweiten Ausführungsbeispiel bildet die Längsachse L die Rotations- und Translationsachse der Komponenten, die an der Dosierung und Produktausschüttung beteiligt sind.

Der vordere Gehäuseabschnitt 1, 3 bildet wie bei dem ersten Ausführungsbeispiel eine Gleitführung für das Dosiseinstellglied 39. Zur Ausbildung der Gleitführung stehen eine innere Mantelfläche des Mechanikhalters 3 und eine äußere Mantelfläche des Dosiseinstellglieds 39 miteinander in Gleitkontakt. Das Dosier- und Betätigungselement 32 steht mit einer Innenmantelfläche des Dosiseinstellglieds 39 in einem Eingriff, um die verdrehgesicherte Verbindung zwischen dem Dosiseinstellglied 39 und dem Dosier- und Betätigungselement 32 zu bilden.

In dem zweiten Ausführungsbeispiel weist die Kolbenstange 4 über die Rückzugssperreinrichtung 6 hinaus keine eigene Bremseinrichtung auf. Die Vorderseiten der Sägezähnen der Rückzugssperreinrichtung 6 bilden vielmehr alleine auch die Bremseinrichtung. Die Kolbenstange 4 des zweiten Ausführungsbeispiels kann jedoch durch die Kolbenstange des ersten Ausführungsbeispiels ersetzt werden. Entsprechend wären in diesem Falle durch den Mechanikhalter 3 des zweiten Ausführungsbeispiels auch wenigstens ein Bremselement, vorzugsweise beide Bremselemente des ersten Ausführungsbeispiels auszubilden.

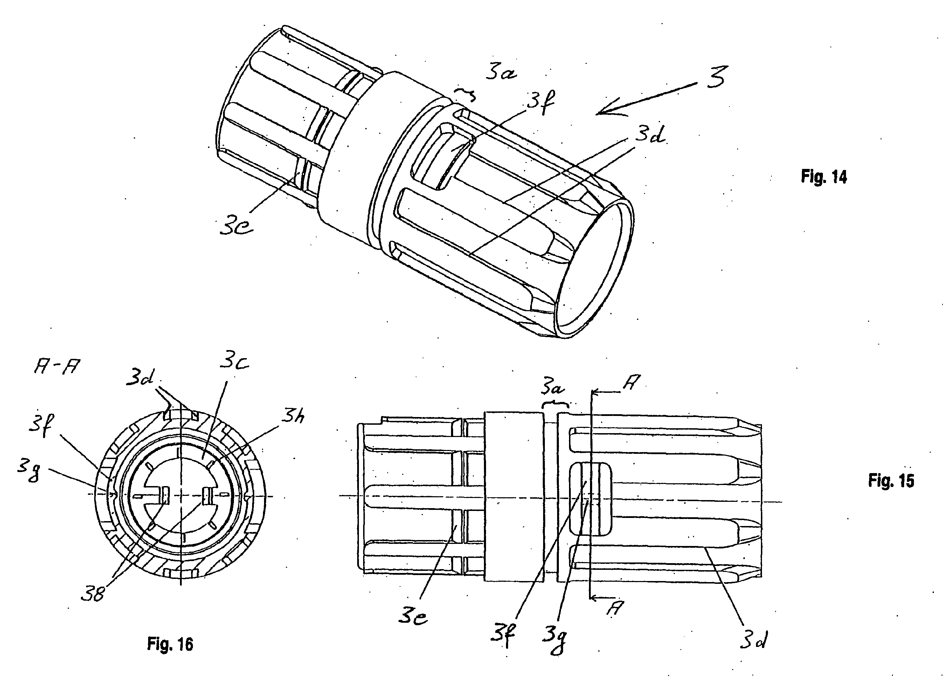

Die Figuren 14 bis 16 zeigen den Mechanikhalter 3 des zweiten Ausführungsbeispiels in einer perspektivischen Darstellung, einer Seitenansicht und in dem in der Seitenansicht eingetragenen Querschnitt A-A. Der Mechanikhalter 3 ist wie beim ersten Ausführungsbeispiel als einteiliges Hülsenteil ausgeführt, vorzugsweise als Kunststoffspritzgussteil. Er weist an dem Außenmantel eines vorderen Hülsenabschnitts einen Wulst 3e auf. Der vordere Hülsenabschnitt wird in das Reservoirteil 1 eingesteckt und mittels des Wulstes 3e zumindest für den Verwender unlösbar mit dem Reservoirteil 1 verrastet.

Das Verriegelungselement 3a ist an einem mittleren Hülsenabschnitt des Mechanikhalters 3 wie bei dem ersten Ausführungsbeispiel ausgebildet.

Ein hinterer Hülsenabschnitt, der sich an das Verriegelungselement 3a anschließt, bildet an seinem Außenumfang eine Mehrzahl von Axialführungen 3d. Die Axialführungen 3d werden von Führungsrippen gebildet, die an dem Außenumfang des hinteren Hülsenabschnitts radial aufragen. Genauer gesagt wird die Axialführung von den axial sich erstreckenden, geraden Seitenwänden dieser Führungsrippen gebildet, so dass, wie bei dem ersten Ausführungsbeispiel, axiale Führungskanäle erhalten werden. Die Führungsrippen ragen von dem mittleren Hülsenabschnitt aus wie Finger bis an das hintere Ende des Mechanikhalters 3, wo sie axial verjüngt auslaufen. Die Axialführung 3d dient der Linearführung des hinteren Gehäuseabschnitts 11 bei dem Verbinden des Reservoirmoduls 10 mit dem Dosier- und Betätigungsmodul 30. Wie in Figur 9 und am besten in Figur 11 zu erkennen ist ragen von einer Innenmantelfläche des hinteren Gehäuseabschnitts 11 der Anzahl nach entsprechend und der Form nach angepasst Eingriffselemente 11 d radial einwärts ab. In jede der Axialführungen 3d ragt ein Eingriffselement 11 d ein und wird von der Axialführung 3d linear geführt, wenn der vordere Gehäuseabschnitt 1, 3 und der hintere Gehäuseabschnitt 11 zum Verbinden ineinander geschoben werden. Auf diese Weise wird sichergestellt, dass zwischen dem vorderen Gehäuseabschnitt 1, 3 und dem hinteren Gehäuseabschnitt 11 keine Relativdrehung stattfinden kann, wenn im Zuge des Verbindens der verdrehgesicherte Eingriff zwischen dem Dosier- und Betätigungselement 32 und dem Dosiseinstellglied 39 hergestellt wird.

Indem die Führungsrippen an ihren hinteren Enden axial verjüngt auslaufen und die Führungskanäle so zu Einführtrichtern verbreitert sind, wird eine Zentrierung zwischen dem vorderen Gehäuseabschnitt 1, 3 und dem hinteren Gehäuseabschnitt 11 zum Zwecke des Verbindens erleichtert. Die Führungsrippen laufen an ihren Enden auch radial zur Mantelfläche des Mechanikhalters 3 verjüngt aus, wodurch die Zentrierung der Gehäuseabschnitte 1, 3 und 11 in eine durch die Axialführung 3d vorgegebene Drehwinkelposition relativ zueinander noch mehr erleichtert wird.

Ebenso wie bei dem Ineinanderschieben der vordere Gehäuseabschnitt 1, 3 und der hintere Gehäuseabschnitt 11 an einer Relativdrehung zueinander gehindert werden, wird auch das Dosiseinstellglied 39 in Bezug auf seine Drehwinkelposition relativ zu dem vorderen Gehäuseabschnitt 1, 3 fixiert, wobei diese Fixierung lösbar ist, um die für die Dosierung erforderliche Drehbewegung des Dosiseinstellglieds 39 zuzulassen. Um daher zum einen die Dosierbewegung des Dosiseinstellglieds 39 zu ermöglichen, aber zum anderen eine unerwünschte Dosierbewegung durch das Herstellen der Verbindung zwischen dem vorderen Gehäuseabschnitt 1, 3 und dem hinteren Gehäuseabschnitt 11 zu verhindern, wird das Dosiseinstellglied 39 von dem Mechanikhalter 3 mittels einer lösbaren Rastverbindung in diskreten Drehwinkelpositionen fixiert.

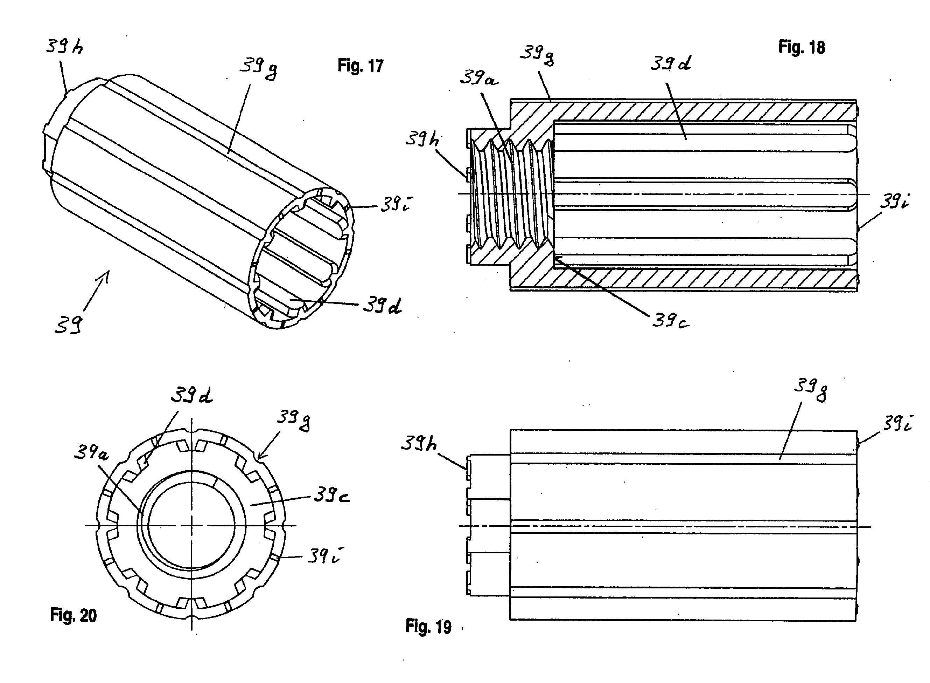

Die Figuren 17 bis 20 zeigen das Dosiseinstellglied 39 in Einzeldarstellungen. Für die Ausbildung der Rastverbindung sind an der Außenmantelfläche des Dosiseinstellglieds 39 über den Umfang in regelmäßiger Teilung verteilt mehrere Rastaufnahmen 39g ausgebildet. Jede der Rastaufnahmen 39g wird von einer geraden, axial sich erstreckenden Rinne mit einer im Querschnitt gerundet verlaufenden Kontur gebildet.

Der Mechanikhalter 3 ist mit zwei Rastnasen 3g (Figur 15 und 16) versehen. Die beiden Rastnasen 3g ragen in dem hinteren Hülsenabschnitt des Mechanikhalters 3 von einer Innenmantelfläche des Mechanikhalters 3 radial einwärts ab. Sie sind einander diametral gegenüberliegend angeordnet. Der jeweilige Mantelbereich des Mechanikhalters 3, an dem eine der Rastnasen 3g angeformt ist, bildet ein in radialer Richtung elastisch nachgiebiges Federelement 3f. Aufgrund der elastischen Nachgiebigkeit und der gerundeten Form der Rastnasen 3g in Verbindung mit dem gerundeten Profil der Rastaufnahmen 39g ist der Rasteingriff der Rastnasen 3g in die gegenüberliegenden Rastaufnahmen 39g lösbar. Die Lösbarkeit ist für die Dosisauswahl erforderlich. Andererseits ist der Rasteingriff jedoch so gestaltet, dass die Drehwinkelfixierung des Dosiseinstellglieds 39 so stabil ist, dass bei dem Verbinden des vorderen Gehäuseabschnitts 1, 3 mit dem hinteren Gehäuseabschnitt 11 keine unerwünschte Dosierbewegung des Dosiseinstellglieds 39 stattfinden kann, wenn die Drehkopplung des Dosier- und Betätigungselements 32 mit dem Dosier- und Betätigungselement 32 hergestellt wird. Die Rastverbindung zwischen dem Mechanikhalter 3 und dem Dosiseinstellglied 39 ergibt als vorteilhaften Nebeneffekt ein taktiles Signal bei der Dosierung. Um die günstige Elastizität des Federelements 3f zu erhalten, ist der hintere Hülsenabschnitt des Mechanikhalters 3 im betreffenden Mantelbereich ausgeschnitten, so dass das Federelement 3f als ein im Umfangsrichtung sich erstreckendes Ringsegment erhalten wird, das axial beidseits freiliegt.

Aus den Figuren 17, 18 und 20 ebenfalls erkennbar sind Axialführungen 39d für den verdrehsicheren Eingriff des Dosiseinstellglieds 39 mit dem Dosier- und Betätigungselement 32. Das Dosier- und Betätigungselement 32 ist mit wenigstens einem Eingriffselement versehen, um die axiale Linearführung, d.h. die Verdrehsicherung, zwischen dem Dosier- und Betätigungselement 32 und dem Dosiseinstellglied 39 zu erhalten. Die Axialführungen 39d sind wieder Führungskanäle, die von mehreren, axial sich gerade erstreckenden Führungsrippen gebildet werden. Jede der Führungsrippen läuft an ihrem hinteren, dem Dosier- und Betätigungselement 32 zugewandten Ende axial und radial verjüngt aus, um die Zentrierung zwischen dem Dosier- und Betätigungselement 32 und dem Dosiseinstellglied 39 bei der Herstellung des verdrehsicheren Eingriffs zu erleichtern. Für die axiale Linearführung von Dosiseinstellglied 39 und Dosier- und Betätigungselement 32 wird somit die gleiche Konstruktion wie für die axiale Linearführung der Gehäuseabschnitte 1, 3 und 11 verwendet.

Der Vollständigkeit wegen sei schließlich auch auf das Dosiergewinde 39a und den Ausschüttanschlag 39c des Dosiseinstellglieds 39 hingewiesen, die am besten in Figur 18 zu erkennen sind.

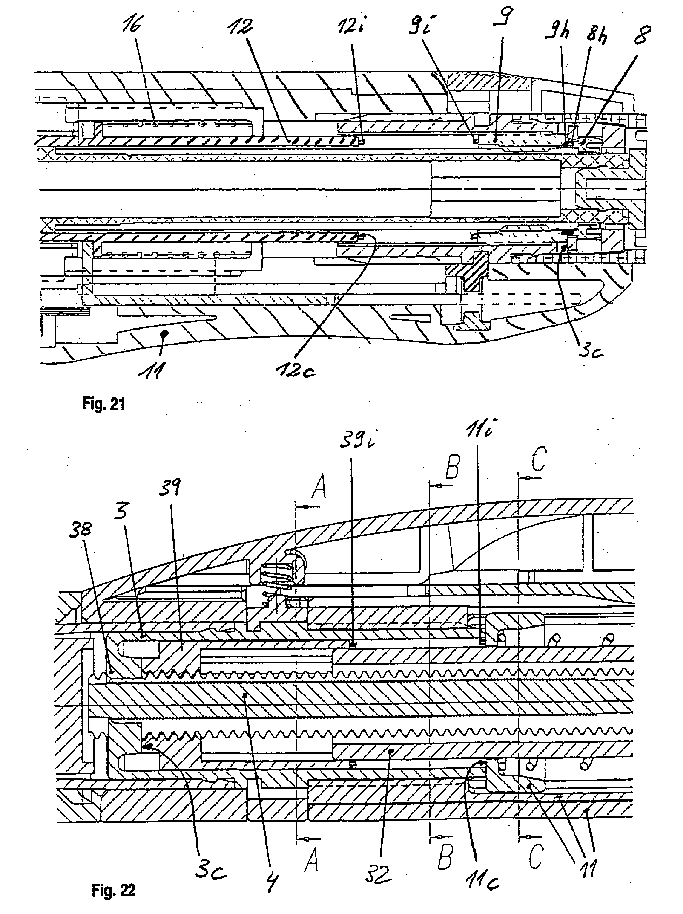

Schließlich sind für das Dosiseinstellglied 39 zwei Verdrehsicherungen vorgesehen, die in den beiden axialen Endpositionen des Dosiseinstellglieds 39 wirksam werden. Diesbezüglich sei ergänzend auf Figur 22 hingewiesen.

Um zu verhindern, dass die Kolbenstange 4 in Reaktion auf eine rotatorische Dosierbewegung des Dosiseinstellglieds 39 zurückbewegt werden könnte, sind an einem vorderen Ende des Dosiseinstellglieds 39 Verdrehanschläge 39h geformt. Die Verdrehanschläge 39h stehen in der vorderen Endposition, die das Dosiseinstellglied 39 unmittelbar nach einer Produktausschüttung bzw. vor einer Dosisauswahl einnimmt, mit Verdrehgegenanschlägen 3h in Eingriff, die an dem Mechanikhalter 3 angeformt sind (Fig. 16). Die Verdrehanschläge 39h ragen von einer vorderen Stirnseite des Dosiseinstellglieds 39 axial ab, und die Verdrehgegenanschläge 3h ragen von einer axial zugewandten Stirnfläche des Mechanikhalters 3, die den Aufschüttanschlag 3c bildet, den Verdrehanschlägen 39h axial entgegen. Der Eingriff zwischen den Verdrehanschlägen 39h und den Verdrehgegenanschlägen 3h ist derart, dass er eine rotatorische Dosierbewegung in eine Drehrichtung zulässt, die eine von dem Ausschüttanschlag 3c weg gerichtete translatorische Dosierbewegung des Dosiseinstellglieds 39 bewirkt, aber in der vorderen axialen Endposition eine rotatorische Dosierbewegung in die Gegendrehrichtung verhindert.

Ferner ist ein weiteres Paar von Verdrehanschlägen und Verdrehgegenanschlägen vorgesehen, die in grundsätzlich gleicher Weise wie die Anschläge 3h und 39h geformt sind und zusammenwirken. Bei diesem zweiten Verdrehanschlagpaar handelt es sich zum einen um Verdrehanschläge 39i, die von einer hinteren Stirnfläche des Dosiseinstellglieds 39 axial abragen, und zum anderen um Verdrehgegenanschläge 11 i, die von der zugewandten Anschlagstirnfläche des hinteren Translationsanschlags 11 c axial auf das Dosiseinstellglied 39 zuragen, in der Figur 9 aufgrund ihrer geringen Abmessungen jedoch nicht erkennbar sind. Durch das hintere Verdrehanschlagpaar 11 i/39i wird in der hinteren Endposition verhindert, dass die Kolbenstange 4 in Reaktion auf eine gegen die Anschlagstirnfläche des hinteren Translationsanschlags 11 c gerichtete Dosierbewegung des Dosiseinstellglieds in Vorschubrichtung bewegt werden kann.
Die Höhe, d.h. die axiale Länge, sämtlicher Verdrehanschläge 3h, 39h, 11i und 39i ist auf die Gewindesteigung der im Eingriff befindlichen Dosiergewinde der Kolbenstange 4 und des Dosiseinstellglieds 39 abgestimmt. Die Verdrehanschläge sind axial so kurz, dass diejenige rotatorische Dosierbewegung nicht behindert wird, durch die das Dosiseinstellglied 39 von dem jeweiligen Translationsanschlag 3c oder 11 c wegbewegt wird.

Bei der Montage der Komponenten des Reservoirmoduls 10 wird das Dosiseinstellglied 39 bis in eine vorgegebene Axialposition, wie sie aus Figur 9 ersichtlich ist, auf die Kolbenstange 4 aufgeschraubt. Anschließend wird die Kolbenstange 4 mit dem aufgeschraubten Dosiseinstellglied 39 von hinten in den Mechanikhalter 3 eingeführt, bis dessen Blockiereinrichtung 38 in Sperreingriff mit der Rückzugssperreinrichtung 6 der Kolbenstange 4 gelangt und ferner der verdrehgesicherte Eingriff zwischen den Verdrehanschlägen 39h des Dosiseinstellglieds 39 und den Verdrehgegenanschlägen 3h des Mechanikhalters 3 hergestellt ist. Bereits während der Einführung in den Mechanikhalter 3 wird das Dosiseinstellglied 39 in einer Drehwinkelrastposition durch den Rasteingriff zwischen den Rastnasen 3g und den Rastaufnahmen 39g von dem Mechanikhalter 3 axial linear geführt, bis das Dosiseinstellglied 39 an den Ausschüttanschlag 3c des Mechanikhalters 3 anstößt. In dieser vorderen Endposition des Dosiseinstellglieds 39 relativ zu dem Mechanikhalter 3 ist gleichzeitig auch bereits der verdrehsichere Eingriff zwischen den Verdrehanschlägen 3h und 39h hergestellt.

In diesem Zustand werden der Mechanikhalter 3 und ein bereits mit einem Reservoir ausgestattetes Reservoirteil 1 miteinander verbunden.

In einem nächsten Schritt wird der hintere Gehäuseabschnitt 11 des komplett montierten Dosier- und Betätigungsmoduls 30 auf den Mechanikhalter 3 geschoben, wobei sich der Mechanikhalter 3 und der hintere Gehäuseabschnitt 11 aufgrund der Axialführungen 3d und der Eingriffselemente 11 d des hinteren Gehäuseabschnitts 11 zueinander zentrieren können und nach der Zentrierung aufgrund des Führungseingriffs axial aneinander linear geführt werden. Im Zuge des Aufschiebens gelangt das Dosier- und Betätigungselement 32 in den verdrehgesicherten Eingriff mit dem Dosiseinstellglied 39, wobei auch hier durch eine den Axialführungen 3d und Eingriffselementen 11 d entsprechende Linearführung zuerst eine gewisse Zentrierung stattfinden kann.

Das Dosier- und Betätigungselement 32 ist in diskreten Drehwinkelrastpositionen mit dem hinteren Gehäuseabschnitt in einem Rasteingriff und wird in dem Rasteingriff, d.h. in der jeweiligen Drehwinkelrastposition, axial linear geführt. Die Drehwinkeldifferenz zwischen zwei aufeinanderfolgenden Drehwinkelrastpositionen entspricht einer Dosiseinheit. Die Linearführung zwischen dem Mechanikhalter 3 und dem hinteren Gehäuseabschnitt 11 einerseits und die diskreten Drehwinkelpositionen des Dosiseinstellglieds 39 relativ zu dem Mechanikhalter 3 (Rastnasen 3g und Rastaufnahmen 39g) und die Drehwinkelrastpositionen des Dosier- und Betätigungselements 32 relativ zu dem hinteren Gehäuseabschnitt 11 andererseits sind aufeinander so abgestimmt, dass ein lineares Übereinanderschieben der beiden Gehäuseabschnitte 1, 3 und 11 stets in solch einer Drehwinkelposition stattfindet, dass auch das Dosiseinstellglied 39 und das Dosier- und Betätigungselement 32 für ihren verdrehgesicherten Eingriff relativ zueinander ausgerichtet sind, so dass während des Verbindens des Reservoirmoduls 10 mit dem Dosier- und Betätigungsmodul 30 keine Relativdrehung zwischen den an der Dosierung beteiligten Komponenten stattfindet.

In Bezug auf die weiteren Details der Montage, insbesondere der Herstellung des Verriegelungseingriffs, und zur Funktionsweise des Injektionsgeräts nach dem zweiten Ausführungsbeispiel wird auf die Beschreibung zu dem ersten Ausführungsbeispiel verwiesen.

Auch bei dem Injekionsgerät nach dem ersten Ausführungsbeispiel können Verdrehsicherungen vorgesehen sein, die in den beiden axialen Endpositionen des Dosiseinstellglieds 9 des ersten Ausführungsbeispiels unerwünschte Reaktionsbewegungen der Kolbenstange 4 verhindern. Figur 21 zeigt die beiden Verdrehsicherungen, die in gleicher Weise wie die Verdrehsicherungen des zweiten Ausführungsbeispiels geformt sind. Allerdings werden die bei dem zweiten Ausführungsbeispiel an den Gehäuseabschnitten 1, 3 und 11 geformten Verdrehgegenanschläge bei dem ersten Ausführungsbeispiel zum einen von der Blockiereinrichtung 8 und zum anderen von dem Dosier- und Betätigungselement 12 gebildet. So sind an der Stirnfläche der Blockiereinrichtung 8, die dem Dosiseinstellglied 9 axial zugewandt ist, mehrere Verdrehanschläge 8h angeformt, die auf das Dosiseinstellglied 9 axial zu ragen. Da die Blockiereinrichtung 8 von dem vorderen Gehäuseabschnitt 1, 3 axial unbeweglich gelagert wird und mit der Kolbenstange 4 verdrehgesichert verbunden ist, wird auch durch das vordere Verdrehanschlagpaar 8h/9h eine Verdrehsicherung für die zwischen der Kolbenstange 4 und dem Dosiseinstellglied 9 stattfindende rotatorische Dosierbewegung erhalten. Das zweite Verdrehanschlagpaar ist zwischen dem Dosiseinstellglied 9 und dem hinteren Translationsanschlag 12c gebildet. Wie bei dem zweiten Ausführungsbeispiel ragen von der Stirnfläche des Translationsanschlags 12c, die dem Dosiseinstellglied 9 axial zugewandt ist, mehrere Verdrehanschläge 12i axial in Richtung auf das Dosiseinstellglied 9 vor. Das Dosiseinstellglied 9 ist wie bei dem zweiten Ausführungsbeispiel an seiner Rückseite mit Verdrehanschlägen 9i versehen, die in der hinteren axialen Endposition des Dosiseinstellglieds 9 in Eingriff mit den Verdrehanschlägen 12i gelangen. In der hinteren axialen Endposition des Dosiseinstellglieds 9 wird durch das hintere Verdrehanschlagpaar 9i/12i nur diejenige rotatorische Dosierbewegung zugelassen, die eine translatorische Dosierbewegung des Dosiseinstellglieds 9 in die Vorschubrichtung bewirkt.

### Bezugszeichen:

- 1: Reservoirteil, Ampullenhalter
- 2: Reservoir, Ampulle
- 3: Mechanikhalter
- 3a: Erstes Verriegelungselement
- 3b: Fixiereinrichtung
- 3c: Ausschüttanschlag, Translationsanschlag
- 3d: Axialführung
- 3e: Wulst
- 3f: Federelement
- 3g: Rastnase
- 3h: Verdrehanschlag
- 4: Kolbenstange
- 4a: Verbindungsabschnitt
- 5: Gewindeabschnitt
- 6: Rückzugsperreinrichtung, Zahnreihe
- 7: Vorschubbremseinrichtung, Zahnreihe
- 8: Blockiereinrichtung
- 8a: Sperrelement
- 8b: Bremselement
- 8h: Verdrehanschlag
- 9: Dosiseinstellglied
- 9h: Verdrehanschlag
- 9i: Verdrehanschlag
- 10: Reservoirmodul
- 11: hinterer Gehäuseabschnitt
- 11d: Eingriffselement
- 11i: Verdrehanschlag
- 12: Dosier- und Betätigungselement
- 12i: Verdrehanschlag
- 13: Dosiermitnehmer
- 14: Verschluss
- 15a: Mitnehmer, Ringsteg
- 15b: Mitnehmer, Ringsteg
- 16: Rückstelleinrichtung
- 17: Zähl- und Anzeigeeinrichtung
- 18: -
- 19: -
- 20: Verriegelungsring
- 21: Zweites Verriegelungselement
- 22: Entriegelungsknopf
- 23: Sperrnocken
- 24: Rückstelleinrichtung
- 25: Verriegelungssperre
- 26: Mitnehmer, Steg
- 27: Axialausnehmung
- 28: Sperrzunge
- 29: Einrückausnehmung
- 30: Dosier- und Betätigungsmodul
- 31: Anschlagelement
- 32: Dosier- und Betätigungselement
- 33-37: -
- 38: Blockiereinrichtung
- 39: Dosiseinstellglied
- 39a: Dosiergewinde
- 39c: Ausschüttanschlag
- 39d: Axialführung
- 39g: Rastaufnahme, Axialführung
- 39h: Verdrehanschlag
- 39i: Verdrehanschlag

## Patentansprüche

1. Verabreichungsgerät für eine dosierte Verabreichung eines fluiden Produkts, das Verabreichungsgerät umfassend:
a) ein Gehäuse (1, 3, 11) mit einem Reservoir (2) für das Produkt,
b) eine Abtriebsvorrichtung (4), die einen Ausschütthub in eine Vorschubrichtung entlang einer Translationsachse (L) ausführt, um eine Produktdosis auszuschütten,
c) eine Antriebsvorrichtung (12; 32), die die Ausschüttbewegung bewirkt,
d) ein Dosiseinstellglied (9; 39), das mit der Abtriebsvorrichtung (4) so gekoppelt ist, dass eine rotatorische Dosierbewegung, die das Dosiseinstellglied (9; 39) und die Abtriebsvorrichtung (4) um die Translationsachse (L) relativ zueinander ausführen, eine axiale translatorische Dosierbewegung des Dosiseinstellglieds (9; 39) relativ zu der Abtriebsvorrichtung (4) und dem Gehäuse (1, 3, 11) bewirkt,
e) einen Translationsanschlag (3c, 12c; 3c, 11c); dem das Dosiseinstellglied (9; 39) in einer axialen Endposition axial zugewandt gegenüberliegt,
f) und eine Verdrehsicherung (8h/9h, 12i/9i; 3h/39h, 11 i/39i), die in der Endposition die rotatorische Dosierbewegung in eine erste Drehrichtung zulässt und in die andere, zweite Drehrichtung blockiert,
**dadurch gekennzeichnet, dass**
g) die Verdrehsicherung (8h/9h, 12i/9i;- 3h/39h, 11i/39i) verhindert, dass das Dosiseinstellglied (9; 39) durch die rotatorische Dosierbewegung axial gegen den Translationsanschlag (3c, 12c; 3c, 11c) gepresst-wird.

2. Verabreichungsgerät nach Anspruch 1, **dadurch gekennzeichnet, dass** die Verdrehsicherung wenigstens einen ersten Verdrehanschlag (39h, 39i) und wenigstens einen zweiten Verdrehanschlag (3h, 11i) umfasst, die in der Endposition des Dosiseinstellglieds (39) gegeneinander auf Anschlag sind, um die rotatorische Dosierbewegung zu verhindern, wobei der wenigstens eine erste Verdrehanschlag (39h, 39i) von dem Dosiseinstellglied (39) und der wenigstens eine zweite Verdrehanschlag (3h,11 i) von dem Gehäuse (1, 3, 11) gebildet oder verdrehgesichert gelagert wird.

3. Verabreichungsgerät nach Anspruch 1, **dadurch gekennzeichnet, dass** die Verdrehsicherung wenigstens einen ersten Verdrehanschlag (9i) und wenigstens einen zweiten Verdrehänschlag (12i) umfasst, die in derEndposition des Dosiseinstellglieds (9) gegeneinander auf Anschlag sind, um die rotatorische Dosierbewegung zu verhindern, wobei der wenigstens eine erste Verdrehanschlag (9i) von dem Dosiseinstellglied (9) und der wenigstens eine zweite Verdrehanschlag (12i) von der Antriebsvorrichtung (12) gebildet oder verdrehgesichert gelagert wird.

4. Verabreichungsgerät nach Anspruch 1, **dadurch gekennzeichnet, dass** die Verdrehsicherung wenigstens einen ersten Verdrehanschlag (9h) und wenigstens einen zweiten Verdrehanschlag (8h) umfasst, die in der Endposition des Dosiseinstellglieds (9) gegeneinander auf Anschlag sind, um die rotatorische Dosierbewegung zu verhindern, wobei der wenigstens eine erste Verdrehanschlag (9h) von dem Dosiseinstellglied (9) gebildet wird und der wenigstens eine zweite Verdrehanschlag (8h) mit der Abtriebsvorrichtung (4) verdrehgesichert verbunden und relativ zu dem Translationsanschlag (3c) axial unbeweglich ist.

5. Verabreichungsgerät nach einem der vorhergehenden Ansprüche, **dadurch gekennzeichnet, dass** die Verdrehsicherung wenigstens einen ersten Verdrehanschlag (9h, 9i; 39h, 39i) und wenigstens einen zweiten Verdrehanschlag (8h, 12i; 3h, 11i) umfasst, die in der Eridposition des Dosiseinstellglieds (9; 39) gegeneinander auf Anschlag sind, um die rotatorische Dosierbewegung zu verhindern, wobei der wenigstens eine erste Verdrehanschlag (9h, 9i; 39h, 39i) und der wenigstens eine zweite Verdrehanschlag (8h, 12i; 3h, 11 i) axial aufeinander zu ragen.

6. Verabreichungsgerät nach einem der vorhergehenden Ansprüche, **dadurch gekennzeichnet dass** die Verdrehsicherung wenigstens einen ersten Verdrehanschlag (9h, 9i; 39h, 39i) und wenigstens einen zweiten Verdrehanschlag (8h, 12i; 3h, 11i) umfasst, die in der Endposition des Dosiseinstellglieds (9; 39) gegeneinander auf Anschlag sind, um die rotatorische Dosierbewegung zu verhindern, wobei der wenigstens eine erste Verdrehanschlag (9h, 9i; 39h, 39i) ein Vorsprung und der wenigstens eine zweite Verdrehanschlag (8h, 12i; 3h, 11i) eine Ausnehmung ist und der Vorsprung für die Blockierung der zweiten rotatorischen Dosierbewegung in die Ausnehmung hineinragt.

7. Verabreichungsgerät nach einem der zwei vorhergehenden Ansprüche, **dadurch gekennzeichnet, dass** der wenigstens eine erste Verdrehanschlag (9h, 9i; 39h, 39i) und der wenigstens eine zweite Verdrehanschlag (8h, 12i; 3h, 11i) je an einer von zwei Stirnflächen gebildet sind, die axial einander zugewandt sind.

8. Verabreichungsgerät nach einem der vorhergehenden Ansprüche, **dadurch gekennzeichnet, dass** die Verdrehsicherung wenigstens einen ersten Verdrehanschlag (9h, 9i; 39h, 39i) und wenigstens einen zweiten Verdrehänschlag (8h, 12i; 3h, 11i) umfässt, die in der Endposition des Dosiseinstellglieds (39) gegeneinander auf Anschlag sind, um die rotatorische Dosierbewegung zu verhindern, wobei der wenigstens eine erste Verdrehanschlag (9h, 9i; 39h, 39i) an dem Dosiseinstellglied (9; 39) und der wenigstens eine zweite Verdrehanschlag (8h; 12i; 3h, 11i) an demwenigstens einen Translationsanschlag (3c, 12c; 3c, 11c) einstückig angeformt ist.

9. Verabreichungsgerät nach einem der vorhergehenden Ansprüche, **dadurch gekennzeichnet, dass** das Dosiseinstellglied (9; 39) ein Gewinde und die Abtriebsvorrichtung (4) ein Gewinde aufweisen und der Eingriff, in dem das Dosiseinstellglied (9; 39) und die Abtriebsvorrichtung (4) sind, ein Gewindeeingriff dieser Gewinde um die Translationsachse (L) ist.

10. Verabreichungsgerät nach einem der vorhergehenden Ansprüche, **dadurch gekennzeichnet, dass** die Verdrehsicherung mehrere erste Verdrehanschläge (9h, 9i; 39h, 39i) und mehrere zweite Verdrehanschläge (8h, 12i; 3h, 11i) umfasst, die in der Endposition des Dosiseinstellglieds (9; 39) paarweise gegeneinander auf Anschlag sind, indem jeder der ersten Verdrehanschläge (9h, 9i; 39h, 39i) mit je einem der zweiten Verdrehanschläge (8h, 12i; 3h, 11i) ein Anschlagpaar bildet, und das die derart gebildeten Anschlagpaare voneinander beabstandet in Umfangsrichtung der rotatorischen Dosierbewegung nebeneinander angeordnet sind.

11. Verabreichungsgerät nach einem der vorhergehenden Ansprüche, **dadurch gekennzeichnet, dass** eine Kanüle von höchstens 30 Gauge, vorzugsweise eine 31 oder 32 Gauge Kanüle, oder eine Kanüle, die eine nicht in ISO 9626 spezifizierte Kombination von Aussendurchmesser und Innendurchmesser aufweist, mit einem Aussendurchmesser von höchstens 320 µm und einer möglichst dünnen Wandstärke eine Injektions- oder Infusionskanüle des Verabreichungsgeräts bildet.

12. Reservoirmodul für das Verabreichungsgerät nach einem der vorhergehenden Ansprüche, das Reservoirmodul (10) umfassend:
a) einen vorderen Gehäuseabschnitt (1, 3) des Verabreichungsgeräts, der ein Reservoir (2) für ein fluides Produkt umfasst,
b) einen Kolben, der in dem Reservoir (2) in eine Vorschubrichtung auf einen Auslass des Reservoirs (2) zu bewegbar aufgenommen ist, um Produkt auszuschütten,
c) eine auf den Kolben wirkende Kolbenstange (4),
d) eine Antriebsvorrichtung (12; 32), die mit der Kolbenstange (4) gekoppelt ist, um die Kolbenstange (4) in die Vorschubrichtung zu bewegen,
e) ein Dosiseinstellglied (9; 39), das mit der Kolbenstange (4) so gekoppelt ist, dass die Kolbenstange (4) und das Dosiseinstellglied (9; 39) einander in die Vorschubrichtung mitnehmen und eine rotatorische Dosierbewegung, die das Dosiseinstellglied (9; 39) und die Kolbenstange (4) um die Translationsachse (L) relativ zueinander ausführen, eine axiale translatorische Dösierbewegung des Dosiseinstellglieds (9; 39) relativ zu der Kolbenstange (4) und dem vorderen Gehäuseabschnitt (1, 3) bewirkt,
**dadurch gekennzeichnet, dass**
f) das Reservoirmodul weiterhin einen Translationsanschlag (3c) aufweist, der die gemeinsame Bewegung der Kolbenstange (4) und des Dosiseinstellglieds (9; 39) in die Vorschubrichtung begrenzt, indem das Dosiseinstellglied (9; 39) in einer vorderen axialen Endposition gegen den Ausschüttanschlag (3c) in Anlage gelangt,
g) und eine Verdrehsicherung (8h/9h, 12i/9i; 3h/39h, 11 li/39i), die in der Endposition die rotatorische Dosierbewegung nur in eine erste Drehrichtung zulässt und in die andere, zweite Drehrichtung blockiert, wobei das Dosiseinsteltglicd (9; 39) durch die zugelassene rotatorische Dosierbewegung von dem Ausschüttanschlag (3c) wegbewegt wird.

13. Reservoirmodul nach dem vorhergehenden Anspruch, **dadurch gekennzeichnet, dass** die Verdrehsicherung wenigstens einen ersten Verdrehanschlag (39h, 39i) und wenigstens einen zweiten Verdrehanschlag (3h, 11i) umfasst, die in der Endposition des Dösiseinstellglieds (39) gegeneinander auf Anschlag sind, um die rotatorische Dosierbewegung zu verhindern, wobei der wenigstens eine erste Verdrehanschlag (39h, 39i) von dem Dosiseinstellglied (39) und der wenigstens eine zweite Verdrehanschlag (3h, 11i) von dem Gehäuse (1, 3, 11) gebildet oder verdrehgesichert gelagert wird.

14. Reservoirmodul nach Anspruch 12, **dadurch gekennzeichnet, dass** die Verdrehsicherung wenigstens einen ersten Verdrehanschlag (9h) und wenigstens einen zweiten Verdrehanschlag (8h) umfasst, die in der Endposition des Dosiseinstellglieds (9) gegeneinander auf Anschlag sind, um die rotatorische Dosierbewegung zu verhindern, wobei der wenigstens eine erste Verdrehanschlag (9h) von dem Dosiseinstellglied (9) gebildet oder verdrehgesichert gelagert wird und der wenigstens eine zweite Verdrehanschlag (8h) mit der Kolbenstange (4) verdrehgesichert verbunden und relativ zu dem Translationsanschlag (3c) axial unbeweglich ist.

15. Reservoirmodul nach dem vorhergehenden Anspruch, **dadurch gekennzeichnet, dass** eine Blockiereinrichtung (8), die von dem vorderen Gehäuseabschnitt (1, 3) axial nicht beweglich, aber um die Translationsachse (L) drehbar gelagert wird und mit der Kolbenstange (4) verdrehgesichert verbunden und in einem Sicherheitseingriff ist, durch den eine Bewegung der Kolbenstange (4) gegen die Vorschubrichtung verhindert oder zumindest erschwert wird, den wenigstens einen zweiten Verdrehanschlag (8h) bildet.

16. Reservoirmodul nach einem der vorhergehenden Ansprüche, **dadurch gekennzeichnet, dass** der vordere Gehäuseabschnitt (1, 3) eine Blockiereinrichtung (8; 38) bildet oder axial nicht bewegbar lagert und dass die Blockiereinrichtung (8; 38) mit der Kolbenstange (4) in einem Sicherheitseingriff ist, um eine Bewegung der Kolbenstange (4) gegen die Vorschubrichtung zu verhindern oder zumindest zu erschweren.

17. Reservoirmodul nach einem der vorhergehenden Ansprüche, **dadurch gekennzeichnet, dass** der vordere Gehäuseabschnitt (1, 3) ein hülsenförmiges Reservoirteil (1) mit dem Reservoir (2) und einen hülsenförmigen Mechanikhalter (3) umfasst, die separat gefertigt und vorzugsweise so miteinander verbunden sind, dass ein Benutzer die Verbindung zerstörungsfrei nicht lösen kann, und das der Mechanikhalter (3) die Kolbenstange (4) hält.

18. Reservoirmodul nach einem der vorhergehenden Ansprüche, **dadurch gekennzeichnet, dass** das Reservoirmodul (10) ein Verbrauchsmodul ist, das nach einer Entleerung des Reservoirs (2) für einen Austausch im Ganzen vorgesehen ist.

## Claims

1. An administering apparatus for administering a fluid product in doses, the administering apparatus comprising:
a) a casing (1, 3, 11) comprising a reservoir (2) for the product;
b) a driven device (4) which performs a delivery stroke in an advancing direction along a translational axis (L), in order to deliver a product dosage;
c) a drive device (12; 32) which effects the delivery movement;
d) a dosage setting member (9; 39) which is coupled to the driven device (4) such that a rotational dosing movement which the dosage setting member (9; 39) and the driven device (4) perform about the translational axis (L) relative to each other causes an axial translational dosing movement of the dosage setting member (9; 39) relative to the driven device (4) and the casing (1, 3, 11);
e) a translational stopper (3c, 12c; 3c, 11c) which the dosage setting member (9; 39) lies opposite, axially facing, in an axial end position;
f) and a rotational block (8h/9h, 12i/9i; 3h/39h, 11i/39i) which in the end position permits the rotational dosing movement in a first rotational direction and blocks the rotational dosing movement in the other, second rotational direction;
**characterised in that**:
g) the rotational block (8h/9h, 12i/9i; 3h/39h, 11i/39i) prevents the dosage setting member (9; 39) from being pressed axially against the translational stopper (3c, 12c; 3c, 11c) by the rotational dosing movement.

2. The administering apparatus according to claim 1, **characterised in that** the rotational block comprises at least one first rotational stopper (39h, 39i) and at least one second rotational stopper (3h, 11i) which abut against each other in the end position of the dosage setting member (39), in order to prevent the rotational dosing movement, wherein the at least one first rotational stopper (39h, 39i) is formed or mounted, secured against rotating, by the dosage setting member (39) and the at least one second rotational stopper (3h, 11i) is formed or mounted, secured against rotating, by the casing (1, 3, 11).

3. The administering apparatus according to claim 1, **characterised in that** the rotational block comprises at least one first rotational stopper (9i) and at least one second rotational stopper (12i) which abut against each other in the end position of the dosage setting member (9), in order to prevent the rotational dosing movement, wherein the at least one first rotational stopper (9i) is formed or mounted, secured against rotating, by the dosage setting member (9) and the at least one second rotational stopper (12i) is formed or mounted, secured against rotating, by the drive device (12).

4. The administering apparatus according to claim 1, **characterised in that** the rotational block comprises at least one first rotational stopper (9h) and at least one second rotational stopper (8h) which abut against each other in the end position of the dosage setting member (9), in order to prevent the rotational dosing movement, wherein the at least one first rotational stopper (9h) is formed by the dosage setting member (9) and the at least one second rotational stopper (8h) is connected, secured against rotating, to the driven device (4) and cannot be moved axially relative to the translational stopper (3c).

5. The administering apparatus according to any one of the preceding claims, **characterised in that** the rotational block comprises at least one first rotational stopper (9h, 9i; 39h, 39i) and at least one second rotational stopper (8h, 12i; 3h, 11i)which abut against each other in the end position of the dosage setting member (9; 39), in order to prevent the rotational dosing movement, wherein the at least one first rotational stopper (9h, 9i; 39h, 39i) and the at least one second rotational stopper (8h, 12i; 3h, 11i) protrude axially towards each other.

6. The administering apparatus according to any one of the preceding claims, **characterised in that** the rotational block comprises at least one first rotational stopper (9h, 9i; 39h, 39i) and at least one second rotational stopper (8h, 12i; 3h, 11i) which abut against each other in the end position of the dosage setting member (9; 39), in order to prevent the rotational dosing movement, wherein the at least one first rotational stopper (9h; 9i; 39h, 39i) is a protrusion and the at least one second rotational stopper (8h, 12i; 3h, 11i) is a recess and the protrusion protrudes into the recess, in order to block the second rotational dosing movement.

7. The administering apparatus according to any one of the preceding two claims, **characterised in that** the at least one first rotational stopper (9h, 9i; 39h, 39i) and the at least one second rotational stopper (8h, 12i; 3h, 11i) are each formed on one of two abutting areas which face each other axially.

8. The administering apparatus according to any one of the preceding claims, **characterised in that** the rotational block comprises at least one first rotational stopper (9h, 9i; 39h, 39i) and at least one second rotational stopper (8h, 12i; 3h, 11i) which abut against each other in the end position of the dosage setting member (39), in order to prevent the rotational dosing movement, wherein the at least one first rotational stopper (9h, 9i; 39h, 39i) is formed as one piece on the dosage setting member (9; 39) and the at least one second rotational stopper (8h, 12i; 3h, 11i) is formed as one piece on the at least one translational stopper (3c, 12c; 3c, 11c).

9. The administering apparatus according to any one of the preceding claims, **characterised in that** the dosage setting member (9; 39) comprises a thread and the driven device (4) comprises a thread, and the engagement between the dosage setting member (9; 39) and the driven device (4) is a threaded engagement of said threads about the translational axis (L).

10. The administering apparatus according to any one of the preceding claims, **characterised in that** the rotational block comprises a number of first rotational stoppers (9h, 9i; 39h, 39i) and a number of second rotational stoppers (8h, 12i; 3h, 11i) which abut against each other in pairs in the end position of the dosage setting member (9; 39), by each of the first rotational stoppers (9h, 9i; 39h, 39i) forming a pair of stoppers with each one of the second rotational stoppers (8h, 12i; 3h, 11i), and wherein the pairs of stoppers formed in this way are arranged adjacently, spaced from each other in the circumferential direction of the rotational dosing movement.

11. The administering apparatus according to any one of the preceding claims, **characterised in that** a cannula of at most 30 gauge, preferably a 31 or 32 gauge cannula, or a cannula exhibiting a combination of outer and inner diameter not specified in ISO 9626, having an outer diameter of 320 µm at most and as thin a wall thickness as possible, forms an injection or infusion cannula of the administering apparatus.

12. A reservoir module for the administering apparatus according to any one of the preceding claims, the reservoir module (10) comprising:
a) a front casing section (1, 3) of the administering apparatus, which comprises a reservoir (2) for a fluid product;
b) a piston which is accommodated in the reservoir (2) such that it can move in an advancing direction towards an outlet of the reservoir (2), in order to deliver product;
c) a piston rod (4) acting on the piston;
d) a drive device (12; 32) which is coupled to the piston rod (4) in order to move the piston rod (4) in the advancing direction;
e) a dosage setting member (9; 39) which is coupled to the piston rod (4) such that the piston rod (4) and the dosage setting member (9; 39) slave each other in the advancing direction, and a rotational dosing movement which the dosage setting member (9; 39) and the piston rod (4) perform relative to each other about the translational axis (L) effects an axial, translational dosing movement of the dosage setting member (9; 39) relative to the piston rod (4) and the front casing section (1, 3);
**characterised in that**:
f) the reservoir module further comprises a translational stopper (3c) which limits the joint movement of the piston rod (4) and the dosage setting member (9; 39) in the advancing direction, by the dosage setting member (9; 39) abutting against the delivery stopper (3c) in a front axial end position;
g) and a rotational block (8h/9h, 12i/9i; 3h/39h, 11/39i) which in the end position permits the rotational dosing movement in a first rotational direction only and blocks the rotational dosing movement in the other, second rotational direction, wherein the dosage setting member (9; 39) is moved away from the delivery stopper (3c) by the permitted rotational dosing movement.

13. The reservoir module according to the preceding claim, **characterised in that** the rotational block comprises at least one first rotational stopper (39h, 39i) and at least one second rotational stopper (3h, 11i) which abut against each other in the end position of the dosage setting member (39), in order to prevent the rotational dosing movement, wherein the at least one first rotational stopper (39h, 39i) is formed or mounted, secured against rotating, by the dosage setting member (39) and the at least one second rotational stopper (3h, 11i) is formed or mounted, secured against rotating, by the casing (1, 3, 11).

14. The reservoir module according to claim 12, **characterised in that** the rotational block comprises at least one first rotational stopper (9h) and at least one second rotational stopper (8h) which abut against each other in the end position of the dosage setting member (9), in order to prevent the rotational dosing movement, wherein the at least one first rotational stopper (9h) is formed or mounted, secured against rotating, by the dosage setting member (9) and the at least one second rotational stopper (8h) is connected, secured against rotating, to the piston rod (4) and cannot be moved axially relative to the translational stopper (3c).

15. The reservoir module according to the preceding claim, **characterised in that** a blocking means (8), which is mounted by the front casing section (1, 3) such that it cannot move axially but can rotate about the translational axis (L), and which is connected, secured against rotating, to the piston rod (4) and is in securing engagement with the piston rod (4), and which prevents the piston rod (4) from moving counter to the advancing direction or at least makes this more difficult, forms the at least one second rotational stopper (8h).

16. The reservoir module according to any one of the preceding claims, **characterised in that** the front casing section (1, 3) forms a blocking means (8; 38) or mounts a blocking means (8; 38) such that it cannot move axially, and **in that** the blocking means (8; 38) is in securing engagement with the piston rod (4), in order to prevent the piston rod (4) from moving counter to the advancing direction or to at least make this more difficult.

17. The reservoir module according to any one of the preceding claims, **characterised in that** the front casing section (1,3) comprises a sleeve-shaped reservoir portion (1) comprising the reservoir (2) and a sleeve-shaped mechanism holder (3) which are produced separately and are preferably connected to each other such that a user cannot release the connection without destroying it, and **in that** the mechanism holder (3) holds the piston rod (4).

18. The reservoir module according to any one of the preceding claims, **characterised in that** the reservoir module (10) is a disposable module which is provided to be exchanged in its entirety once the reservoir (2) has been emptied.

## Revendications

1. Appareil distributeur pour une distribution dosée d'un produit fluide, l'appareil distributeur comprenant :
a) un boîtier (1, 3, 11) avec un réservoir (2) pour le produit,
b) un dispositif d'évacuation (4) qui effectue une course d'évacuation le long d'un axe de translation (L) dans une direction d'avancement pour extraire une dose de produit,
c) un dispositif d'entraînement (12 ; 32) qui engendre le mouvement d'évacuation,
d) un élément de réglage des doses (9 ; 39) qui est couplé avec le dispositif d'évacuation (4) de telle manière qu'un mouvement tournant de dosage que réalisent l'un par rapport à l'autre l'élément réglage des doses (9 ; 39) et le dispositif d'évacuation (4) autour de l'axe translation (L) engendre un mouvement de translation axial de dosage de l'élément de réglage des doses (9 ; 39) par rapport au dispositif d'évacuation (4) et au boîtier (1, 3, 11),
e) une butée de blocage en translation (3c, 12c ; 3c, 11c) en face de laquelle se trouve dirigé dans le sens axial l'élément réglage des doses (9 ; 39) dans une position d'extrémité axiale et
f) un système de blocage en rotation (8h/9h, 12i/9i; 3h/39h, 11i/39i) qui, dans la position d'extrémité, permet le mouvement tournant de dosage dans un premier sens de rotation et le bloque dans l'autre et deuxième sens de rotation,
**caractérisé en ce que**
g) le système de blocage en rotation (8h/9h, 12i/9i ; 3h/39h, 11i/39i) évite que l'élément de réglage des doses (9 ; 39) soit poussé dans le sens axial contre la butée de blocage en translation (3c, 12c ; 3c, 11c) par le mouvement tournant de dosage.

2. Appareil distributeur selon la revendication 1, **caractérisé en ce que** le système de blocage en rotation comprend au moins une première butée de blocage en rotation (39h, 39i) et au moins une deuxième butée de blocage en rotation (3h, 11i) qui sont en butée l'une contre l'autre dans la position d'extrémité de l'élément de réglage des doses (39) pour éviter le mouvement tournant de dosage, ladite au moins une première butée de blocage en rotation (39h, 39i) étant formée sur ou solidarisée avec un blocage en rotation à l'élément de réglage des doses (39) et ladite au moins une deuxième butée de blocage en rotation (3h, 11i) étant formée sur ou solidarisée avec un blocage en rotation au boîtier (1, 3, 11).

3. Appareil distributeur selon la revendication 1, **caractérisé en ce que** le système de blocage en rotation comprend au moins une première butée de blocage en rotation (9i) et au moins une deuxième butée de blocage en rotation (12i) qui sont en butée l'une contre l'autre dans la position d'extrémité de l'élément de réglage des doses (9) pour éviter le mouvement tournant de dosage, ladite au moins une première butée de blocage en rotation (9i) étant formée sur ou solidarisée avec un blocage en rotation à l'élément de réglage des doses (9) et l'au moins une deuxième butée de blocage en rotation (12i) étant formée sur ou solidarisée avec un blocage en rotation au dispositif d'entraînement (12).

4. Appareil distributeur selon la revendication 1, **caractérisé en ce que** le système de blocage en rotation comprend au moins une première butée de blocage en rotation (9h) et au moins une deuxième butée de blocage en rotation (8h) qui sont en butée l'une contre l'autre dans la position d'extrémité de l'élément de réglage des doses (9) pour éviter le mouvement tournant de dosage, ladite au moins une première butée de blocage en rotation (9h) étant formée sur l'élément de réglage des doses (9) et ladite au moins une deuxième butée de blocage en rotation (8h) étant reliée avec un blocage en rotation avec le dispositif d'évacuation (4) et n'étant pas mobile dans le sens axial par rapport à la butée de blocage en translation (3c).

5. Appareil distributeur selon l'une quelconque des revendications précédentes, **caractérisé en ce que** le système de blocage en rotation comprend au moins une première butée de blocage en rotation (9h, 9i ; 39h, 39i) et au moins une deuxième butée de blocage en rotation (8h, 12i ; 3h, 11i) qui sont en butée l'une contre l'autre dans la position d'extrémité de l'élément de réglage des doses (9 ; 39) pour éviter le mouvement tournant de dosage, ladite au moins une première butée de blocage en rotation (9h, 9i; 39h, 39i) et ladite au moins une deuxième butée de blocage en rotation (8h, 12i ; 3h, 11i) s'avançant l'une vers l'autre dans le sens axial.

6. Appareil distributeur selon l'une quelconque des revendications précédentes, **caractérisé en ce que** le système de blocage en rotation comprend au moins une première butée de blocage en rotation (9h, 9i ; 39h, 39i) et au moins une deuxième butée de blocage en rotation (8h, 12i ; 3h, 11i) qui sont en butée l'une contre l'autre dans la position d'extrémité de l'élément de réglage des doses (9 ; 39) pour éviter le mouvement tournant de dosage, ladite au moins une première butée de blocage en rotation (9h, 9i ; 39h, 39i) étant une saillie et ladite au moins une deuxième butée de blocage en rotation (8h, 12i ; 3h, 11i) étant un évidement et la saillie pénétrant dans l'évidement pour assurer le blocage du deuxième mouvement tournant de dosage.

7. Appareil distributeur selon l'une quelconque des deux revendications précédentes, **caractérisé en ce que** ladite au moins une première butée de blocage en rotation (9h, 9i ; 39h, 39i) et ladite au moins une deuxième butée de blocage en rotation (8h, 12i ; 3h, 11 i) sont formées respectivement sur une surface frontale parmi deux, lesquelles sont dirigées l'une vers l'autre dans le sens axial.

8. Appareil distributeur selon l'une quelconque des revendications précédentes, **caractérisé en ce que** le système de blocage en rotation comprend au moins une première butée de blocage en rotation (9h, 9i ; 39h, 39i) et au moins une deuxième butée de blocage en rotation (8h, 12i ; 3h, 11i) qui sont en butée l'une contre l'autre dans la position d'extrémité de l'élément de réglage des doses (39) pour éviter le mouvement tournant de dosage, ladite au moins une première butée de blocage en rotation (9h, 9i ; 39h, 39i) étant formée d'une seule pièce sur l'élément de réglage des doses (9 ; 39) et ladite au moins une deuxième butée de blocage en rotation (8h, 12i ; 3h, 11i) étant formée d'une seule pièce sur ladite au moins une butée de blocage en translation (3c, 12c ; 3c, 11c).

9. Appareil distributeur selon l'une quelconque des revendications précédentes, **caractérisé en ce que** l'élément de réglage des doses (9 ; 39) comprend un filet et le dispositif d'évacuation (4) comprend un filet et la prise, dans laquelle sont l'élément de réglage des doses (9 ; 39) et le dispositif d'évacuation (4), est un vissage de ces filets autour de l'axe de translation (L).

10. Appareil distributeur selon l'une quelconque des revendications précédentes, **caractérisé en ce que** le système de blocage en rotation comprend plusieurs premières butées de blocage en rotation (9h, 9i ; 39h, 39i) et plusieurs deuxièmes butées de blocage en rotation (8h, 12i ; 3h, 11i) qui, par le fait que chacune des premières butées de blocage en rotation (9h, 9i ; 39h, 39i) forme une paire de butées avec respectivement une des deuxièmes butées de blocage en rotation (8h, 12i ; 3h, 11i), sont en butée l'une contre l'autre par paires dans la position d'extrémité de l'élément de réglage des doses (9 ; 39) et **en ce que** les paires de butées ainsi formées sont disposées l'une à côté de l'autre dans la direction périphérique du mouvement tournant de dosage en étant espacées l'une de l'autre.

11. Appareil distributeur selon l'une quelconque des revendications précédentes, **caractérisé en ce qu'**une canule de jauge maximum 30, de préférence une canule de jauge 31 ou 32, ou une canule qui présente une combinaison, entre le diamètre extérieur et le diamètre intérieur, non spécifiée dans ISO 9626, avec un diamètre extérieur d'au maximum 320 µm et une épaisseur de paroi la plus faible possible, forme une canule d'injection ou d'infusion de l'appareil distributeur.

12. Module de réservoir pour l'appareil distributeur selon l'une quelconque des revendications précédentes, le module de réservoir (10) comprenant :
a) une section de boîtier avant (1, 3) de l'appareil distributeur qui comprend un réservoir (2) pour un produit fluide,
b) un piston qui est disposé dans le réservoir (2) en étant déplaçable dans une direction d'avancement vers une sortie du réservoir (2) pour extraire du produit,
c) une tige de piston (4) agissant sur le piston,
d) un dispositif d'entraînement (12 ; 32) qui est couplé avec la tige de piston (4) pour déplacer la tige de piston (4) dans la direction d'avancement,
e) un élément de réglage des doses (9 ; 39) qui est couplé avec la tige de piston (4) de telle façon que la tige de piston (4) et l'élément de réglage des doses (9 ; 39) s'entraînent mutuellement dans la direction d'avancement et qu'un mouvement tournant de dosage que l'élément de réglage des doses (9 ; 39) et la tige de piston (4) effectuent l'un par rapport à l'autre autour de l'axe de translation (L) engendre un mouvement de dosage axial de translation de l'élément de réglage des doses (9 ; 39) par rapport à la tige de piston (4) et à la section de boîtier avant (1, 3),
**caractérisé en ce que**
f) le module de réservoir comprend par ailleurs une butée de blocage en translation (3c) qui, par le fait que l'élément de réglage des doses (9 ; 39) arrive dans une position d'extrémité axiale avant en appui contre la butée d'évacuation (3c), limite le mouvement commun de la tige de piston (4) et de l'élément de réglage des doses (9 ; 39) dans la direction de l'avancement et
g) comprend un système de blocage en rotation (8h/9h, 12i/9i ; 3h/39h, 11i/39i) qui, dans la position d'extrémité, ne permet le mouvement tournant de dosage que dans un premier sens de rotation et le bloque dans l'autre et deuxième sens de rotation, l'élément de réglage des doses (9 ; 39) étant déplacé à l'écart du dispositif d'évacuation (3c) par le mouvement tournant de dosage autorisé.

13. Module de réservoir selon la revendication précédente, **caractérisé en ce que** le système de blocage en rotation comprend au moins une première butée de blocage en rotation (39h, 39i) et au moins une deuxième butée de blocage en rotation (3h, 11i) qui sont en butée l'une contre l'autre dans la position d'extrémité de l'élément de réglage des doses (39) pour éviter le mouvement tournant de dosage, ladite au moins une première butée de blocage en rotation (39h, 39i) étant formée sur ou étant solidarisée avec un blocage en rotation à l'élément de réglage des doses (39) et ladite au moins une deuxième butée de blocage en rotation (3h, 11i) étant formée sur ou étant solidarisée avec un blocage en rotation au boîtier (1, 3, 11).

14. Module de réservoir selon la revendication 12, **caractérisé en ce que** le système de blocage en rotation comprend au moins une première butée de blocage en rotation (9h) et au moins une deuxième butée de blocage en rotation (8h) qui sont en butée l'une contre l'autre dans la position d'extrémité de l'élément de réglage des doses (9) pour éviter le mouvement tournant de dosage, ladite au moins une première butée de blocage en rotation (9h) étant formée sur ou étant solidarisée avec un blocage en rotation à l'élément de réglage des doses (9) et ladite au moins une deuxième butée de blocage en rotation (8h) étant reliée avec un blocage en rotation avec la tige de piston (4) et n'étant pas mobile dans le sens axial par rapport à la butée de blocage en translation (3c).

15. Module de réservoir selon la revendication précédente, **caractérisé en ce qu'**un dispositif de blocage (8) qui est solidarisé de manière non déplaçable dans le sens axial mais de manière à tourner autour de l'axe de translation (L) à la section de boîtier avant (1, 3) et qui est relié avec un blocage en rotation avec la tige de piston (4) en étant avec celle-ci dans une prise de sécurité par laquelle un mouvement de la tige de piston (4) contre la direction d'avancement est évité ou au moins rendu plus difficile, forme ladite au moins une deuxième butée de blocage en rotation (8h).

16. Module de réservoir selon l'une quelconque des revendications précédentes, **caractérisé en ce que** la section de boîtier avant (1, 3) forme ou loge de manière non déplaçable dans le sens axial un dispositif de blocage (8 ; 38) et **en ce que** le dispositif de blocage (8 ; 38) est en prise de sécurité avec la tige de piston (4) pour éviter ou au moins pour rendre plus difficile un mouvement de la tige de piston (4) contre la direction d'avancement.

17. Module de réservoir selon l'une quelconque des revendications précédentes, **caractérisé en ce que** la section de boîtier avant (1, 3) comprend une partie de réservoir en forme de douille (1) renfermant le réservoir (2), et un support mécanique en forme de douille (3), lesquels sont fabriqués séparément et sont de préférence reliés entre eux de telle façon qu'un utilisateur ne peut pas désolidariser la liaison sans provoquer sa destruction, et le support mécanique (3) maintient la tige de piston (4).

18. Module de réservoir selon l'une quelconque des revendications précédentes, **caractérisé en ce que** le module de réservoir (10) est un module consommable qui est prévu pour un échange complet lorsque le réservoir (2) est vide.
